# EUROPÄISCHE PATENTSCHRIFT

(11) **EP 3 790 515 B1**
(45) Veröffentlichungstag und Bekanntmachung des Hinweises auf die Patenterteilung: **01.04.2026**
(21) Anmeldenummer: 19723086.5
(22) Anmeldetag: 08.05.2019
(51) Int. Cl.: A61F 2/72, A61N 1/04, A61B 5/389

(54) **VERFAHREN ZUM EINRICHTEN EINES MYOELEKTRISCH GESTEUERTEN PROTHESENSYSTEMS UND PROTHESENSYSTEM**
METHOD FOR SETTING UP A MYOELECTRIC-CONTROLLED PROSTHESIS SYSTEM AND PROSTHESIS SYSTEM
PROCÉDÉ DE CONFIGURATION D'UN SYSTÈME DE PROTHÈSE COMMANDÉ MYOÉLECTRIQUEMENT ET SYSTÈME DE PROTHÈSE

(30) Priorität: 09.05.2018 DE 102018111241
(43) Veröffentlichungstag der Anmeldung: 17.03.2021
(60) Teilanmeldung: 26158872.7
(73) Patentinhaber: Otto Bock Healthcare Products GmbH, 1110 Wien (AT)
(72) Erfinder: AMSÜSS, Sebastian, 1180 Wien (AT); MESSNER, Christoph, 1060 Wien (AT); BRANDMAYR, Georg, 1210 Wien (AT); WEHRLE, Martin, 1070 Wien (AT); FRIEDRICH, Markus, 3424 Muckendorf (AT); STEININGER, Johannes, 2154 Gaubitsch (AT)
(74) Vertreter: Gramm, Lins & Partner Patent- und Rechtsanwälte PartGmbB
(86) Internationale Anmeldenummer: PCT/EP2019/061796
(87) Internationale Veröffentlichungsnummer: WO 2019/215214

(56) Entgegenhaltungen:
- WO-A1-2018/026842
- CN-A- 1 582 866
- DE-U1- 202006 007 460
- RU-C1- 2 635 632
- US-B1- 8 591 599
- US-B1- 8 828 093

## Beschreibung

Die Erfindung betrifft ein Verfahren gemäß Anspruch 1 zum Einrichten eines myoelektrisch gesteuerten Prothesensystems mit einem Prothesenschaft und mehreren Ableiterelektroden zur Erfassung elektrischer Muskelaktivitäten. Die Erfindung betrifft ebenfalls ein Prothesensystem gemäß Anspruch 15 mit einem Prothesenschaft, der an einem Gliedmaßenstumpf anlegbar ist mit mehreren Ableiterelektroden zur Erfassung elektrischer Muskelaktivitäten, einer mit den Ableiterelektroden verbundenen Steuerungseinheit und einer angetriebenen Prothesenkomponente, die an dem Prothesenschaft befestigt und mit der Steuerungseinheit gekoppelt ist.

Prothesen dienen zum Ersatz einer fehlenden Gliedmaße. Neben rein optischen Aspekten ist es wünschenswert, dass die Prothese möglichst viele Funktionen der nicht mehr vorhandenen Gliedmaße ausführen kann, um dem Patienten Erleichterungen bei den Tätigkeiten des alltäglichen Lebens zu bieten. Bei Prothesen der unteren Extremität werden häufig Prothesenkomponenten über einen Prothesenschaft an einer verbliebenen Gliedmaße oder einem Gliedmaßenstumpf festgelegt. Dies kann auf unterschiedliche Art und Weise geschehen, beispielsweise über ein Gurtsystem oder über ein Unterdrucksystem und/oder ein Linersystem mit mechanischen Verriegelungselementen an dem distalen Ende des Liners. An dem Prothesenschaft ist häufig distal ein Gelenk angeordnet, um eine weitere Prothesenkomponente gelenkig anzubinden, beispielsweise ein Prothesenkniegelenk oder ein Prothesenknöchelgelenk. Bei Prothesen der unteren Extremität können die Gelenke als Sperrgelenke, einmalig hinsichtlich ihres Dämpfungsverhaltens eingestellte Gelenke, sensorgesteurte passive Gelenke mit über den jeweiligen Bewegungsablauf anpassbarem Dämpfungsverhalten oder auch als motorisch angetriebene Gelenke ausgebildet sein.

Auch bei oberen Extremitäten reichen die Ausgestaltungen der Prothesen von reinen Schmuckprothesen über Seilzug aktivierte Greifeinrichtungen bis zu motorisch angetriebenen Prothesenhänden, die an Oberarmschäften über ein Ellenbogengelenk an dem Patienten befestigt sind.

Bei angetriebenen Prothesen ist es notwendig, die Antriebe präzise anzusteuern, damit die jeweilige Prothesenkomponente die gewünschte oder erforderliche Bewegung ausführt. Die Steuerung kann nach Art einer Fernbedienung über eine gesunde Gliedmaße erfolgen. Dazu werden Sensoren an einer gesunden Gliedmaße angeordnet und Bewegungssignale aufgenommen. Jedem Sensorsignal wird ein Steuerungsbefehl für den jeweiligen Antrieb zugeordnet. Wird eine bestimmte Bewegung ausgeführt und ein Sensorsignal detektiert, wird über die in einem Rechner abgelegte Steuerung ein Befehl an den Antrieb übermittelt, eine bestimmte Verlagerung oder Verschwenkung über einen bestimmten Weg in einer bestimmten Zeit auszuführen.

Alternativ besteht die Möglichkeit, über Elektroden Biosignale eines Patienten aufzunehmen, beispielsweise elektrische Muskelaktivität aufgrund von Muskelkontraktionen. Diese Signale werden als elektromyografische Signale aufgenommen und können zur Steuerung eines Antriebes oder mehrerer Antriebe verwendet werden. Über erlernte Kontraktionsmuster ist ein Patient in der Lage, durch die Kontraktion eines Muskels oder mehrere Muskeln unterschiedliche Aktionen der Prothesenkomponente ausführen zu lassen. Hierfür reichen häufig zwei Elektroden aus, über die eine sogenannte Zweikanal-Versorgung stattfinden kann. Eine Greifeinrichtung kann über eine einfache Signalfolge leicht geöffnet und geschlossen werden.

Komplexe prothetische Versorgungen, beispielsweise mit einem angetriebenen Prothesenellenbogengelenk an einer Prothesenhand mit einzeln verlagerbaren Prothesenfingern und/oder einem flektierbaren und/oder rotierbaren Handgelenk, sind mit einer Zweikanalsteuerung nicht alltagstauglich durch einen Patienten zu steuern. Zur Steuerung einer solchen komplexen Versorgung werden acht oder mehr Elektrodenpaare benötigt, um über eine Mustererkennung eine Zuordnung vieler myoelektrischer Signale vornehmen und daraus eine entsprechend komplexe Steuerung ableiten zu können.

Patienten sollten nach dem Verlust einer Gliedmaße möglichst schnell eine prothetische Versorgung erhalten, um die noch vorhandenen Muskeln optimal zu nutzen und um darüber hinaus zu vermeiden, dass sich ein Patient an den Zustand ohne Gliedmaße adaptiert. Problematisch hierbei kann sein, dass bei Amputationen die Wunde noch verheilen muss und dass Schwellungen oder Quetschungen auskuriert werden müssen, so dass das Anpassen eines endgültigen Prothesenschaftes nicht möglich ist. Darüber hinaus ist es bei einem neu mit einer Prothese zu versorgenden Patienten schwierig vorherzusagen, welche Art der Versorgung überhaupt möglich ist.

Die US 2014/0031952 A1 betrifft Verfahren und Systeme, um physiologische Vorrichtungen und ein prothetisches System miteinander zu verbinden. Dazu werden eine Vielzahl unterschiedlicher Arten physiologischer Aktivitätssignale von dem Nutzer empfangen und auf Grundlage eines jeden Signaltyps eine Bewegungsabsicht dekodiert. Die Bewegungsabsichten werden zu einer gemeinsamen Entscheidung gebündelt, um bewegliche Elemente der Prothesenvorrichtung zu steuern.

Die DE 10 2009 056 466 A1 betrifft ein System und Verfahren zur adaptiven Steuerung und Regelung einer Prothese mit einer Willkürsteuerung, wobei zunächst an einem Muskel eines Prothesenträgers eine Vielzahl von Muskelaktivitätssignalen gemessen werden. Gleichzeitig werden Zustandsinformationen zu einem tatsächlichen Bewegungszustand des Prothesenträgers gemessen, aus denen mindestens ein möglicher, aktueller Bewegungszustand bestimmt wird. Danach wird aus den Muskelaktivitätssignalen mit Hilfe eines Verfahrens zur Detektion von Signalmustern Muskelaktivitätsmerkmale extrahiert. Aus den extrahierten Muskelaktivitätsmerkmalen wird mit Hilfe des aktuellen Bewegungszustandes mindestens ein Willkürsignal bestimmt. Das Willkürsignal wird zur Auswertung und/oder zur Regelung und/oder zur Steuerung einer Aktorik der Prothese verwendet.

Die US 2009/0216339 A1 betrifft ein System zum Durchleiten myoelektrischer Signale durch einen Liner einer prothetischen Vorrichtung. Ein flexibler leitender Elektrodeneinsatz definiert einen ersten Bereich, der an einer inneren Oberfläche des Lines dergestalt angeordnet ist und die Haut des Nutzers berührt. Ein zweiter Bereich erstreckt sich durch den Liner, ein dritter Bereich erstreckt sich an der äußeren Oberfläche des Liners. Über einen Kleber werden zumindest der zweite und dritte Bereich des Einsatzes an dem Liner festgelegt.

Die DE 10 2008 036 714 A1 betrifft ein Verfahren zur Visualisierung mehrkanaliger Sensorsignale, insbesondere myoelektrischer Signale, die über Elektroden von einer Gliedmaße oder einem Amputationsstumpf abgeleitet werden. Jedem Sensorsignal werden eine Anzeigerichtung und ein Anzeigebetrag zugeordnet. Die Anzeigerichtung repräsentiert genau ein Sensorsignal, der Anzeigebetrag repräsentiert die Intensität des jeweiligen Signals. Die Anzeigerichtung und der Anzeigebetrag aller Sensorsignale werden gleichzeitig in Echtzeit auf einem Anzeigegerät als ein graphisches Objekt dargestellt.

Die WO 2018/026842 A1 betrifft eine Technologie zur Signalverarbeitung, mit der beabsichtigte Gliedmaßenbewegungen von intramuskulären oder oberflächenelektromyographischen Signalen abgeleitet werden. Elektromyographische Signale werden von einem Elektrodenarray erfasst und zu einem Prozessor, der mit einem Speicher verbunden ist, geleitet. Die elektromyographischen Daten sind mit einer Trainingsbewegung assoziiert, die von einem Nutzer als Antwort auf eine Trainingsbewegungsaufforderung ausgeführt wird. Aus den single-ended-Kanälen werden Differenzialkanalpaarungen festgelegt und charakteristische Datenmerkmale extrahiert. Die charakteristischen Datenmerkmale werden der Bewegungsaufforderung zugeordnet. Ein Satz charakteristischer Datenmerkmale wird als ein Trainingsinput in einem Dekodiermodell ausgewählt, das konfiguriert ist, um den charakteristischen Merkmalsdatensatz mit der beabsichtigten Bewegung zu korrelieren. Das Dekodiermodell wird so konfiguriert, dass es das charakteristische Datenmodell verwendet.

Die US 8 591 599 B1 betrifft eine Elektrodenanordnung zu Erfassung von Muskelsignalen in einem Prothesenliner, bei der die Signale über Kabel und Stecker weitergeleitet werden.

Die US 8 828 093 B1 betrifft eine myoelektrisch gesteuerte Prothese der unteren Extremität, bei der die Gangphase ermittelt und ein Bewegungsmuster darauf basierend festgelegt wird. Es werden allgemein Domelektroden erwähnt.

Die DE 20 2006 007 460 U1 betrifft ein Prothesenschaft-System mit einem Innenschaft und einem Außenschaft, wobei in dem Innenschaft zumindest eine Ausnehmung zur Durchleitung myoelektrischer Signale einer an dem Innenschaft gelagerten Elektrode vorhanden ist. Die Elektrode ist in einer die Ausnehmung dichtend abschließenden Halterung befestigt.

Die CN 1 582 688 A1 betrifft eine Prothesenhand und eine Steuerung über myoelektrische Signalmuster. Eine Erregung einer in einem Prothesenschaft festgelegten Elektrode ermöglicht eine haptische oder sensorische Rückkopplung, um Unfälle zu vermeiden.

Die RU 2 635 632 C1 betrifft ein Verfahren zur Steuerung einer Prothese einer oberen Extremität, bei der myoelektrische Signale als Reaktion auf eine vorgegebene Bewegung erfasst werden. Diese Signale werden aufbereitet und mit Attributen versehen, die auf der Amplitude der Signale beruhen. Auf der Grundlage der Attribute wird die jeweils gewünschte Bewegung angesteuert. Der Nutzer erhält ein Feedback, darüber hinaus wird die patientengestützte Steuerung durch eine automatische Steuerung ergänzt.

Aufgabe der vorliegenden Erfindung ist es, ein Verfahren zum Einrichten eines myoelektrisch gesteuerten Prothesensystems und einem Prothesensystem als solches bereitzustellen, mit dem möglichst schnell ermittelt werden kann, welche Versorgung für den Patienten möglich ist und wie das Prothesensystem am besten konfiguriert werden kann.

Erfindungsgemäß wird diese Aufgabe durch ein Verfahren mit den Merkmalen des Anspruchs 1 und ein Prothesensystem mit den Merkmalen des Anspruchs 15 gelöst. Vorteilhafte Ausgestaltungen und Weiterbildungen der Erfindung sind in den abhängigen Ansprüchen offenbart.

Das Verfahren zum Einrichten eines myoelektrisch gesteuerten Prothesensystems mit einem Prothesenschaft und mehreren Ableiterelektroden zur Erfassung elektrischer Muskelaktivitäten sieht zunächst das Anliegen einer Oberflächenelektrodenanordnung von mehreren Oberflächenelektroden um den Umfang eines Gliedmaßenstumpfes vor. Über die Oberflächenelektroden der Oberflächenelektrodenanordnung wird elektrische Muskelaktivität an Muskeln des Gliedmaßenstumpfes myoelektrische Signale erfasst. Die myoelektrischen Signale werden hinsichtlich ihrer Signalqualität ausgewertet. Auf Grundlage der Auswertung der Signalqualität wird eine Auswahl des Steuerungsverfahrens getroffen, mit dem das Prothesensystem gesteuert wird. Anschließend werden die Ableiterelektroden an dem Prothesenschaft festgelegt. Über die Oberflächenelektrodenanordnung ist es möglich, ohne dass ein Prothesenschaft bereits endgültig mit Ableiterelektroden ausgestattet sein muss, myoelektrische Signale aufzunehmen und hinsichtlich einer Signalqualität auszuwerten. Unter Signalqualität wird unter anderem die Trennbarkeit der Signale verstanden, insbesondere die Gestalt oder der Verlauf des Signals oder der Ort, an dem das Signal aufgenommen wird. Weiterhin ist die Wiederholbarkeit des Signals ein Qualitätskriterium, eine hohe Signalqualität ist erreicht, wenn das Signal immer gleich aussieht, wenn ein Patient also immer das gleiche Signal erzeugen kann. Auch die Aufrechterhaltbarkeit des Signals ist für die Signalqualität relevant, wenn das Signal für einen längeren Zeitraum erzeugt und abgenommen werden kann ist dies besser als nur ein kurz andauerndes Signal, das ggf. als eine Störgröße aufgefasst und nicht berücksichtigt werden könnte. Dazu werden die Patienten mit der angelegten Oberflächenelektrodenanordnung aufgefordert, Muskelaktivitäten auszuführen, beispielsweise diejenige Tätigkeit auszuführen, die die Protheseneinrichtung ausführen soll, beispielsweise Hand öffnen, Hand schließen, Handgelenk drehen oder die Hand flektieren. Selbstverständlich kann diese Tätigkeit nicht unmittelbar von den Patienten ausgeführt werden, da die durch die Prothese ersetzte Gliedmaße fehlt, jedoch können die verbliebenen Muskeln wie vor dem Verlust der Gliedmaße aktiviert werden. Dadurch entstehen elektrische Potentiale, die durch Oberflächenelektroden aufgenommen und zu einer Auswerteeinrichtung oder Steuerungseinrichtung übermittelt werden. Anhand beispielsweise der Signaldauer, der Signalintensität, der Flankensteigung oder auch einer Signalfrequenz bei Muskelkontraktionen ist es möglich, eine Unterscheidung zu treffen ob eine komplexe elektronische Steuerung einer Protheseneinrichtung überhaupt möglich ist. Diese Eigenschaften der Signale sind Bestandteil der Signalqualität, die zur Auswertung und zur Entscheidung über die Art der Steuerung und die Art der prothetischen Versorgung verwendet wird. Sind beispielsweise Muskeln und/oder Nerven so stark geschädigt, dass keine komplexen, ausreichend eindeutigen Signale von den Patienten erzeugt werden können, wird eine Auswahl der Versorgung des Patienten dahingehend getroffen, dass eine Zweikanal-Versorgung die optimale Versorgung darstellt. Ist ein Patient in der Lage eine ausreichende Anzahl an myoelektrischen Signalen in einer ausreichenden Qualität mit einer ausreichenden Unterscheidbarkeit zu erzeugen, kann über eine Mustererkennung oder eine andere Auswertung der myoelektrischen Signale eine komplexe prothetische Versorgung mit einer Vielzahl von Antrieben und Funktionen durch den Patienten willentlich gesteuert werden. Ein solcher Patient kann dann mit einer entsprechenden Prothese mit Prothesenschaft, angetriebenen Prothesenkomponenten, Steuerungseinrichtungen und dergleichen ausgestattet werden. Auf Grundlage der Informationen darüber, ob eine Zweikanal-Versorgung oder eine Mehrkanalversorgung sinnvoll ist und ausgeführt wird, werden dann die Ableiterelektroden, die ebenfalls als Oberflächenelektroden ausgebildet sein können, an der entsprechenden Stelle des endgültigen Prothesenschaftes festgelegt. Dadurch wird erreicht, dass die ausgewählte Steuerung für die Antriebe der Protheseneinrichtung stets Signale aus den vorab bestimmten Muskelregionen oder von den jeweiligen Muskeln erhält, wodurch sich die Steuerungsgenauigkeit und damit die Qualität der Ausführung der Bewegungen durch die Prothesenkomponente erhöht.

Bevorzugt ist die Oberflächenelektrodenanordnung unabhängig von dem Prothesenschaft ausgebildet und wird separat von dem Prothesenschaft angelegt, um über den Umfang verteilt die Oberflächenelektroden an dem Stumpf der Gliedmaße anzuordnen. Die Oberflächenelektroden werden auf die Hautoberfläche des Gliedmaßenstumpfes, an dem die Prothese angeordnet werden soll, aufgebracht, ohne dass es notwendig ist, eine mehr oder weniger komplizierte Abformung des Gliedmaßenstumpfes zur Anfertigung eines Positiv-Modells vorzunehmen, auf dem dann der Prothesenschaft aus faserverstärkten Kunststoffen aufmodelliert wird. Grundsätzlich sind auch andere Herstellverfahren für einen Prothesenschaft möglich, ebenso wie die Verwendung anderer Materialien möglich und vorgesehen ist. Die Anordnung der Oberflächenelektroden separat von dem Prothesenschaft über die Oberflächenelektrodenanordnung erhöht die Flexibilität der Positionierung und ermöglicht zudem eine sehr frühe Untersuchung und Bestimmung, ob und welche elektromyografischen Signale ein Patient erzeugen kann.

Bevorzugt wird die Oberflächenelektrodenanordnung verschieblich und/oder verdrehbar an den Gliedmaßenstumpf angelegt, um im angelegten Zustand die Oberflächenelektrodenanordnung an unterschiedlichen Stellen des Gliedmaßenstumpfes anzuordnen. Durch das Verdrehen und/oder Verschieben der Oberflächenelektrodenanordnung auf dem Gliedmaßenstumpf können die optimalen Positionen der Elektroden erfasst werden. Die Oberflächenelektrodenanordnung wird solange verdreht und/oder verschoben, bis die bestmöglichen Signale aufgrund von Muskelkontraktionen durch den Patienten erzeugt und erfasst werden können. Die bestmöglichen Signale sind insbesondere diejenigen Signale, die hinsichtlich ihrer Signalqualität insgesamt am besten für die Steuerung geeignet sind. Dazu werden alle Signale betrachtet, so dass eine Vielzahl von Parametern betrachtet und ausgewertet werden müssen. Die Bewertung kann beispielsweise ergeben, dass in einer ersten Stellung zwar die Amplitude zweier Signal nicht maximal ist, jedoch die Flankensteilheit und die räumliche Trennung der Signale besser als in einer zweiten Stellung mit maximalen Amplituden, so dass die erste Stellung trotz nicht maximaler Werte in den Kriterien die beste Stellung oder Position ist.

Bevorzugt werden die Oberflächenelektroden gleichmäßig oder im Wesentlichen gleichmäßig zueinander beabstandet um den Gliedmaßenstumpf herum angeordnet, bei sechs Elektroden beispielsweise in einem Abstand von 60° oder ungefähr 60° zueinander, bei acht Elektroden in einem Abstand von 45° oder ungefähr 45° zueinander und bei vier Elektroden in einem Abstand von 90°oder ungefähr 90° zueinander. Die myoelektrischen Signale der Oberflächenelektroden werden bevorzugt hinsichtlich ihrer Singnalqualität, insbesondere ihrer Amplitude, ihrer Dauer, ihrer Frequenz und/oder ihrer Bandbreite ausgewertet. Die Auswertung erfolgt hinsichtlich ihrer Verwendbarkeit zur Identifikation von Befehlen. Wird beispielsweise erkannt, dass überhaupt nur zwei Muskel in der Lage sind, verwertbare myoelektrische Signale zu erzeugen, kann eine Versorgung mit einer prothetischen Einrichtung ausgeschlossen werden, die auf einer vielkanaligen Steuerung beruht, beispielsweise zu einer Steuerung an der Prothesenhand mit multiplen Antrieben.

Eine Weiterbildung des Verfahrens sieht vor, dass eine Auswahl und eine Festlegung einer Grundeinstellung des Steuerungsverfahrens vorgenommen werden, beispielsweise die Grundeinstellung dahingehend, dass eine Zweikanalsteuerung oder eine Mustererkennung vorgenommen wird oder eine Grundeinstellung für einen Orthopädietechniker, der diese dann an den jeweiligen Patienten anpasst. Die Auswahl des Steuerungsverfahrens kann automatisch in einem Controller oder einem Computer auf Grundlage vorab definierter Kriterien getroffen werden, insbesondere auf Grundlage der Auswertbarkeit der myoelektrischen Signale. In einer Weiterbildung wird als Steuerungsverfahren eine Mustererkennung ausgewählt und eine ausschließlich von einer autorisierten Person modifizierbare und löschbare Grundeinstellung des Mustererkennungsverfahrens vorgenommen.

Eine Weiterbildung sieht vor, dass eine auf Basis der ermittelten myoelektrischen Signale eines Patienten personalisierte oder kalibrierte Steuerung als Grundeinstellung, die von dem Patienten nicht veränderbar ist, gespeichert und in dem Controller oder dem Computer abgelegt wird. Eine Weiterbildung der Erfindung sieht vor, dass die beiden am besten getrennten myoelektrischen Signale, die von den Oberflächenelektroden aufgenommen werden, identifiziert und als Ausgangspunkt für eine Entscheidung verwendet werden, welche Steuerungsart überhaupt verwendet wird. Diese beiden besten Signale werden als Leitsignal eingesetzt.

Eine Weiterbildung der Erfindung sieht vor, dass die Oberflächenanordnung auf dem Gliedmaßenstumpf in eine Endstellung verschoben und/oder verdreht wird, wobei in der Endstellung zumindest zwei myoelektrische Signale detektierbar sind. Die Detektierbarkeit ist beispielsweise gegeben, wenn ein ausreichend großer Abstand hinsichtlich der vorab definierten Kriterien für die Signalqualität vorhanden ist. Die Ableiterelektroden, die in dem Prothesenschaft fixiert werden, werden dann korrespondierend zu der Endstellung an dem Prothesenschaft festgelegt. Die Anordnung der Elektroden an dem Prothesenschaft erfolgt dann dergestalt, dass die Ableiterelektroden diejenigen Positionen einnehmen, die die entsprechenden Oberflächenelektroden auf der Oberflächenanordnung innegehabt haben, als die optimale Position der Oberflächenelektrodenanordnung beziehungsweise der Oberflächenelektroden auf dem Gliedmaßenstumpf detektiert und über die Auswertung bestimmt worden ist.

Hierzu kann ein in Segmente unterteiltes Band verwendet werden, mit dem es möglich ist, die jeweiligen Positionen an dem Prothesenschaft anzuzeichnen, damit Durchtrittsöffnungen in den Schaft eingebracht werden können.

Eine Weiterbildung der Erfindung sieht vor, dass die Ableiterelektroden über eine Domverschraubung an dem Prothesenschaft festgelegt werden. Bei einer Versorgung eines Patienten muss ein Orthopädietechniker einen Prothesenschaft mit einer Vielzahl von Ableiterelektroden als Einrichtungen zur Erfassung myoelektrischer Signale ausstatten. Eine gegenwärtig durchaus übliche Versorgung sieht eine Achtkanalversorgung vor, bei der zumindest 16 Elektrodenkontakte, gegebenenfalls noch mehrere zusätzliche Elektrodenkontakte für die Erdung an dem Prothesenschaft, beispielsweise einem Innenschaft, verschraubt werden müssen. Dabei ist es wichtig, die Ableiterelektroden mit einem geringen Montageaufwand, einer geringen Bauhöhe und einer hohen Qualität an dem jeweils gewünschten Ort festlegen zu können. Aus dem Stand der Technik sind sogenannte Saugschaftelektroden bekannt, die einen aktiven Verstärker mit einem Gehäuse und integrierten Kontakten aufweisen. Diese Saugschaftelektroden müssen in eine Aussparung in dem Innenschaft befestigt werden. Sollen die Saugschaftelektroden versetzt werden, muss eine entsprechend große Aussparung in einem neuen Innenschaft eingebracht werden, die Lage des Verstärkers muss bereits bei dem Anfertigen des Innenschaftes definiert sein. In einem alternativen Verfahren wird nach dem Bohren eines Loches in dem Prothesenschaft eine Gewindestange eines Domes durch das Loch gesteckt. Eine Unterlegscheibe wird auf die Gewindestange aufgesetzt und eine Mutter angezogen. Eine Kontaktöse einer Elektrode wird über die Gewindestange gesteckt und über eine weitere Mutter festgelegt. Mit zwei Schraubenschlüsseln werden die beiden Muttern gegeneinander verspannt. Abschließend werden überstehende Gewindestangen abgeschliffen. Die Vielzahl der verwendeten Komponenten erschwert die Montage und bringt eine Vergleichsweise große Aufbauhöhe. Das Abschleifen der Gewindestange bewegt das Gewinde, wodurch ein Umplatzieren der Elektroden erschwert wird. Eine elektrische Isolierung nach außen in Richtung Außenschaft ist nicht vorhanden, sodass bei einer Ausgestaltung eines Prothesenaußenschaftes aus einem Carbonmaterial Störungen auftreten können. Durch die erfindungsgemäße Verwendung einer Domverschraubung wird die Montage wesentlich vereinfacht. Nach der Einbringung eines Loches in dem Schaft wird eine Domelektrode mit einem Gewinde durch das Loch gesteckt und über eine Dommutter, die mit einer feuchtigkeitsdichtenden Unterlegscheibe und einer elektrischen Kontaktierung versehen sind, verschraubt. Dies erfolgt über ein einziges Werkzeug, beispielsweise einen Sechskantschlüssel, einen Vierkantschlüssel oder beispielsweise einen einfachen Schraubendreher. Anschließend wird eine Abdeckkappe auf die Dommutter aufgeklipst, um eine elektrische Isolierung nach außen zu bewirken. Dadurch wird eine wesentlich geringere Aufbauhöhe erreicht. Es wird nur noch ein einziges Werkzeug benötigt. Der Dom mit dem Gewinde ist vorteilhafterweise pilzförmig ausgebildet und weist auf der Unterseite des Kopfes ggf. Vorsprünge oder Einrichtungen auf, die eine Verdrehung an der Oberfläche des Innenschaftes oder des Prothesenschaftes verhindern oder erschweren. Dadurch entfällt die Notwendigkeit eines zweiten Werkzeuges, um den Dom zu sichern. Die Unterlegscheibe sichert die Dommutter gegenüber dem Dom gegen ein unbeabsichtigtes Lösen. Darüber hinaus kann ein Dichtring an der Außenseite der Dommutter vorgesehen sein, der weiterhin eine Sicherung gegen unbeabsichtigtes Lösen und gegen Feuchtigkeitsdurchtritt bereitstellt. Nach der Montage werden keine weiteren Kürzungen einer Gewindestange notwendig, die verwendeten Dome und Dommuttern können problemlos wiederverwendet werden. Bevorzugt sind die Dome aus einem nichtrostenden Material, beispielsweise Titan, ausgebildet.

Eine Weiterbildung der Erfindung sieht vor, dass die Ableiterelektroden über Kabel mit einer Steuerungseinheit oder einem Controller verbunden und mechanisch über ein Sicherungselement gegen ein Lösen gesichert werden. Die Steuerungseinheit weist Steckeraufnahmen auf, in die Stecker von den jeweiligen Ableiterelektroden, die fest an dem Prothesenschaft montiert sind, eingesteckt werden können. Um ein Lösen der Stecker im Betrieb der Prothese zu verhindern, wird das Sicherungselement über der Steckverbindung an der Steuerungseinheit befestigt, beispielsweise elastisch über das Gehäuse der Steuerungseinheit geklipst, sodass die Steckerrückseiten gegen das Sicherungselement anliegen und die Kabel durch Ausnehmungen innerhalb des Sicherungselementes hindurch reichen. Das Sicherungselement kann alle Stecker gemeinsam sichern.

Das Prothesensystem mit einem Prothesenschaft, die an einem Gliedmaßenstumpf anlegbar ist, mit mehreren Ableiterelektroden zur Erfassung elektrischer Muskelaktivitäten, einer mit den Ableiterelektroden verbundenen Steuerungseinheit und einer angetriebenen Prothesenkomponente, die an den Prothesenschaft befestigt und mit der Steuerungseinheit gekoppelt ist, sieht eine Oberflächenelektrodenanordnung mit mehreren Oberflächenelektroden vor, die um den Umfang eines Gliedmaßenstumpfes anlegbar sind, wobei die Oberflächenelektrodenanordnung verschieblich und/oder verdrehbar an dem Gliedmaßenstumpf anordenbar ist. Die Steuerungseinheit ist über zumindest einen Stecker mit der oder den Ableiterelektrode(n) oder den Oberflächenelektroden lösbar verbunden. Zwischen der Steuerungseinheit und dem Stecker ist ein Adapter zur Umwandlung myoelektrischer Signale, deren Qualität und Unterscheidbarkeit für eine Mehrkanalsteuerung geeignet sind, in Zweikanalsteuerungssignale angeordnet.

Durch die verschiebliche und/oder verdrehbare Ausgestaltung der Oberflächenelektrodenanordnung ist es möglich, die grundsätzliche Eignung eines Patienten für bestimmte Prothesensysteme oder Steuerungseinrichtungen und Ausgestaltungen eines Prothesensystems zu bestimmen. Anders als bei einer festen Installation von Ableiterelektroden an einem Prothesenschaft, beispielsweise einem Protheseninnenschaft, ist die Oberflächenelektrodenanordnung unabhängig von dem Prothesenschaft verschiebbar und/oder verdrehbar an dem Gliedmaßenstumpf anordenbar. Die Oberflächenelektroden auf der Oberflächenelektrodenanordnung können dann mühelos die elektrischen Muskelaktivitäten erfasst und als myoelektrische Signale weiterverarbeitet werden. Statt einen Patienten zu trainieren, bestimmte Muskeln auf eine bestimmte Art und Weise anzuspannen, kann durch die Oberflächenelektrodenanordnung auf einfache Art und Weise eine optimale Positionierung der Oberflächenelektroden und anschließend der Ableiterelektroden detektiert werden. Die Oberflächenelektroden auf der Oberflächenelektrodenanordnung werden in ihrer Ausrichtung zu dem Gliedmaßenstumpf erfasst und die als Optimum erkannte Position der Oberflächenelektrodenanordnung an dem Gliedmaßenstumpf und damit auch der Oberflächenelektroden auf der Oberfläche des Gliedmaßenstumpfes wird anschließend auf dem Prothesenschaft übertragen, der die Ableiterelektroden an den gleichen Stellen im angelegten Zustand positioniert und der Prothesenschaft ist in der Regel als ein individuell an den Gliedmaßenstumpf angepasster Prothesenschaft ausgebildet, sodass eine Verdrehung oder eine Verschiebung relativ zu dem Gliedmaßenstumpf nicht möglich ist. In der einmal festgelegten Position des Prothesenschaftes an dem Gliedmaßenstumpf sind die Ableiterelektroden als definitive Elektroden an denjenigen Positionen angeordnet, die mittels der Oberflächenelektrodenanordnung als die jeweils beste Position identifiziert worden ist.

Vorteilhafterweise ist die Oberflächenelektrodenanordnung als eine Manschette ausgebildet, die um den Gliedmaßenstumpf herum angelegt werden kann. Die Oberflächenelektrodenanordnung oder Manschette kann als geschlossenes, ringförmiges Band ausgebildet sein oder ein solches Trägerelement aufweisen, wobei sie bevorzugt in Umfangsrichtung elastisch ausgebildet ist. Neben einer Ausgestaltung der Oberflächenelektrodenanordnung oder Manschette oder des Trägerelementes aus einem elastischen Gewebe kann diese oder dieses auch aus einem elastischen Kunststoff, beispielsweise aus einem Kunststoff in Wellenform ausgebildet sein, wobei die Wellenform oder Wellenkontur im Querschnitt vorzugsweise die Oberflächenelektroden auf den nach innen gerichteten Vorsprüngen oder den Bereichen mit dem geringsten Abstand zum Mittelpunkt des Querschnittes oder zur Mittelachse in Längserstreckung, also in Proximal-Distalerstreckung der Oberflächenelektrodenanordnung, aufweisen, sodass die Oberflächenelektroden stets einen guten Kontakt mit der Hautoberfläche des Gliedmaßenstumpfes haben können.

Vorzugsweise sind die Oberflächenelektroden in Umfangsrichtung gleichmäßig oder im Wesentlichen gleichmäßig zueinander beabstandet an der Oberflächenelektrodenanordnung angeordnet, sodass die Signale in einem Diagramm gleichmäßig aufgetragen werden können. An der Oberflächenelektrodenanordnung, z.B. an dem Trägerelement, können Markierungen oder Kennzeichnungen angeordnet sein, die die jeweiligen Positionen der Oberflächenelektroden identifizieren, beispielsweise können die Positionen fortlaufend nummeriert oder mit fortlaufenden Buchstaben versehen sein.

Insbesondere bei einer Ausgestaltung der Oberflächenanordnung als ringförmige oder bandartige Manschette oder mit einem ringförmigen oder bandartigen Trägerelement mit einem distalen Ende und einem proximalen Ende und zumindest einer proximalen Einführöffnung ist es vorteilhaft, wenn die Oberflächenelektrodenanordnung zumindest ein sich in Proximal-/Distalrichtung erstreckendes Stabilisierungselement aufweist, um eine Verkürzung in Proximal-/Distalrichtung bei einer Aufweitung in Umfangsrichtung zu verhindern. Die Stabilisierungselemente können stabartig ausgebildet sein und vorteilhafterweise die Oberflächenelektroden aufnehmen.

An der Oberflächenelektrodenanordnung kann ein auswechselbarer Überzug angeordnet sein, der vorzugsweise aus einem dehnbaren Material, beispielsweise einem dehnbaren Textil besteht. An der Oberflächenelektrodenanordnung, z.B. an dem Trägerelement und/oder an dem Überzug können Markierungen angeordnet sein, um die richtige Zuordnung sowohl der Oberflächenelektrodenanordnung zu den Elektroden als auch des Überzugs zu der Oberflächenelektrodenanordnung und den Elektroden vornehmen zu können. Der Überzug kann auf der Oberflächenelektrodenanordnung über Formschlusselemente festgelegt sein, beispielsweise über Klettverschluss, alternativ können Knöpfe, Haken o.ä. an der Oberflächenelektrodenanordnung vorgesehen sein, um daran den Überzug zu befestigen. Innerhalb des Überzuges sind vorteilhafterweise Ausnehmungen für die Oberflächenelektroden ausgebildet, um einen unmittelbaren Hautkontakt der Oberflächenelektroden mit der Hautoberfläche des Gliedmaßenstumpfes zu ermöglichen. Die Ausnehmungen verhindern gleichzeitig eine Relativverlagerung des Überzuges zu der Oberflächenelektrodenanordnung während der Benutzung, da die nach innen hervorstehenden Oberflächenelektroden eine Verdrehung oder eine Verschiebung des Überzuges verhindern.

Da die Oberflächenelektrodenanordnung als eine Systemkomponente ausgebildet ist, die unabhängig von dem Prothesenschaft an dem Gliedmaßenstumpf anlegbar ist und nur bevorzugt zum Einrichten oder zum Konfigurieren des Prothesensystems verwendet wird, besteht für die Oberflächenelektrodenanordnung bei einer endgültigen Ausgestaltung des Prothesenschaftes mit fixierten Ableiterelektroden keine Verwendung mehr. Daher kann die Oberflächenelektrodenanordnung dann dem Orthopädietechniker zurückgegeben werden, der dann den Überzug entsorgt und die übrige Oberflächenelektrodenanordnung nach vorschriftsmäßiger Reinigung einem weiteren Patienten mit einem neuen Überzug zur Verfügung stellen kann.

In einer Variante der Erfindung ist es vorgesehen, dass die Oberflächenanordnung in dem definitiven Prothesenschaft integriert und festgelegt wird und die Oberflächenelektroden die Ableiterelektroden ausbilden, die dann die myoelektrischen Signale für die Steuerung der angetriebenen Protheseneinrichtung liefert.

Die Oberflächenelektroden und/oder Ableiterelektroden können als Domschraube mit Dommutter ausgebildet sein und mit der Oberflächenelektrodenanordnung oder dem Prothesenschaft verschraubt sein. Über die Ausgestaltung der jeweiligen Elektroden als Domschrauben mit Dommuttern, die durch eine Durchtrittsöffnung in der Oberflächenelektrodenanordnung, beispielsweise innerhalb der Manschette oder des Trägerelementes, oder in dem Prothesenschaft ausgebildet sind, kann eine einfache, schnelle und mit nur einem Werkzeug auszuführende Befestigung einer Vielzahl von Elektroden in der richtigen Position durchgeführt werden. Über die Ausgestaltung der Elektroden als Domschraube mit Dommutter kann eine feuchtigkeitsdichte Befestigung der Elektroden erreicht werden. Die Domschraube kann einen Hinterschnitt aufweisen, über den eine hohe Flächenpressung in dem umgebenden Bereich erreicht wird, sodass Feuchtigkeit nicht hinter die kappenartige Auflagefläche der Domschraube gelangen kann. Darüber hinaus kann an der Außenseite der Dommutter ein Dichtring angeordnet sein, der die Dommutter gegenüber der Durchtrittsöffnung abdichtet. Auf der Außenseite der Oberflächenelektrodenanordnung oder des Prothesenschaftes kann eine zusätzliche Dichtscheibe in Verbindung mit einer elektrischen Kontaktierung vorhanden sein, sodass insgesamt eine feuchtigkeitsdichte Befestigung der Elektroden an dem jeweiligen Träger erreicht werden kann.

Eine Weiterbildung sieht vor, dass zumindest ein Kabel lösbar an dem Prothesenschaft oder der Oberflächenelektrodenanordnung elektrisch kontaktierend mit der Ableiterelektrode oder der Oberflächenelektrode befestigt ist. Gegenüber der Oberflächenanordnung oder dem Prothesenschaft ist das Kabel isoliert, ebenso ist die jeweilige Elektrode gegenüber der Umgebung isoliert, sodass keine Signalstörung auftreten kann. Dadurch ist es möglich, dass nur über das Kabel, das elektrisch mit der Elektrode kontaktiert ist, die myoelektrischen Signale zu der jeweiligen Steuerungseinheit geleitet werden können.

Eine mechanische Sicherung gegen ein Lösen entgegen der Steckrichtung des Steckers ist in Gestalt eines Sicherungselementes in einer Weiterbildung vorgesehen, in der beispielsweise in dem Sicherungselement eine Ausnehmung für das jeweilige Kabel vorgesehen ist. Die Ausnehmung ist so bemessen, dass die Rückseite des Steckers gegen das Sicherungselement anliegt, sodass bei der sicheren Befestigung des Sicherungselementes an der Steuerungseinrichtung, beispielsweise über eine elastische Schnappeinrichtung oder ein Formschlusselement, eine mechanische Sicherung des jeweiligen Kabels erreicht wird.

Die Beschreibung offenbart ebenfalls eine Oberflächenelektrodenanordnung, insbesondere in Gestalt einer Manschette, wie sie vorstehend beschrieben worden ist. Die Beschreibung offenbart ebenfalls eine Elektrode in Gestalt einer Oberflächenelektrode oder einer Ableiterelektrode, wie sie vorstehend beschrieben worden ist. Die Beschreibung offenbart ebenfalls ein Sicherungselement als solches, wie es vorstehend beschrieben worden ist.

Nachfolgend werden Ausführungsbeispiele der Erfindung anhand der beigefügten Figuren näher erläutert.

Es zeigen:
- Figur 1 -: eine Gesamtansicht einer Ableiterelektrode;
- Figur 2-: eine Schnittdarstellung einer Ableiterelektrode vor der Montage;
- Figur 3 -: eine Schnittdarstellung einer Ableiterelektrode im montierten Zustand;
- Figur 4 -: eine perspektivische Darstellung einer Oberflächenelektrodenanordnung mit Oberflächenelektroden;
- Figur 5 -: eine Querschnittsansicht der Oberflächenelektrodenanordnung gemäß Figur 4;
- Figur 6 -: eine Oberflächenelektrodenanordnung mit einem auswechselbaren Überzug;
- Figur 7 -: eine Positionierhilfe;
- Figur 8 -: eine Variante einer Positionierhilfe;
- Figur 9 -: einen Ablauf des Anlegens einer Oberflächenelektrodenanordnung;
- Figur 10 -: eine Steuerungseinheit mit einem Sicherungselement ohne Stecker;
- Figur 11 -: einen Ablauf der Montage von Steckern an einer Steuerungseinheit mit Sicherungselement;
- Figur 12: eines schematische Darstellung eines Prothesensystems; sowie
- Figur 13: eine Teilansicht einer fertiggestellten Prothese.

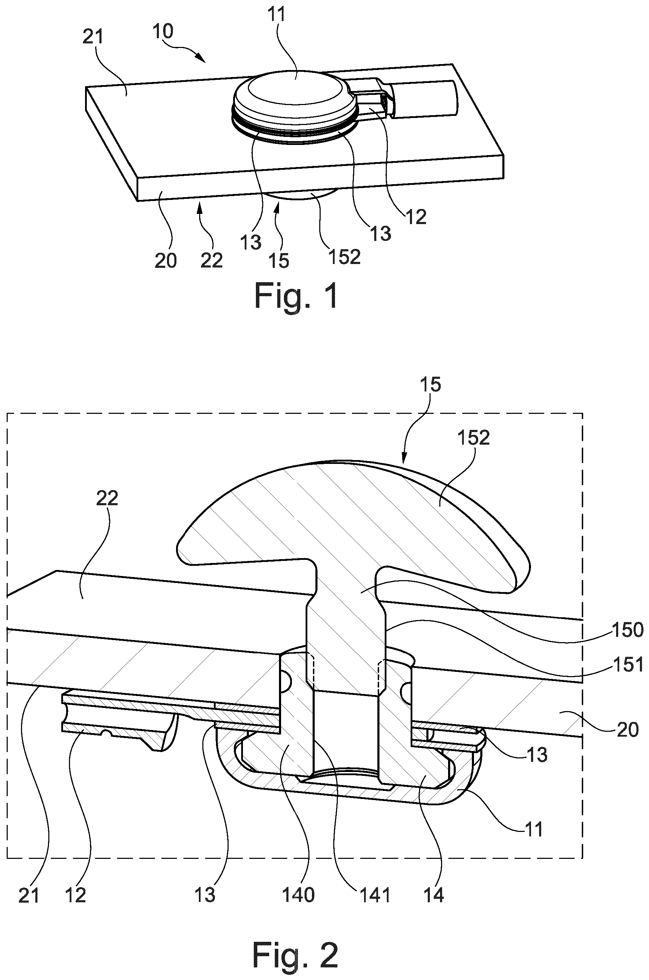

Figur 1 zeigt in einer perspektivischen Darstellung eine montierte Elektrode in Gestalt einer Ableiterelektrode 10, die an einem Grundkörper in Gestalt eines Prothesenschaftes 20 montiert ist. Auf der einem Patienten abgewandten Außenseite 21 ist die Ableiterelektrode 10 mit einer isolierenden Abdeckkappe 11 abgedeckt, die verhindert, dass Signale, die von der Hautoberfläche oder von dem Patienten grundsätzlich aufgenommen werden, zu nicht vorgesehenen Stellen geleitet werden oder durch Störsignale beeinträchtigt werden. Die Abdeckkappe 11 ist um eine Kontaktlasche 12 herum angeordnet, die Kontaktlasche 12 ist wiederum zwischen zwei Scheiben 13 angeordnet, die einerseits eine elektrische Isolierung gegenüber dem Prothesenschaft 20 und darüber hinaus eine Abdichtung einer Durchtrittsstelle durch den Prothesenschaft 20 bewirken. Die Kontaktlasche 12 ist zwischen den beiden Scheiben 13 eingeklemmt, die Scheiben 13 weisen ebenso wie die Kontaktlasche 12 eine Durchtrittsöffnung auf, durch die eine nicht zu erkennende Dommutter hindurch gesteckt wird. Auf einer dem Patienten zugewandten Innenseite 22 wird die Dommutter mit einer Domschraube 15 verschraubt, die einen Schaft mit einem Außengewinde und einen Kopf, den sogenannten Dom 152 aufweist. Der Dom tritt in unmittelbaren Kontakt mit der Hautoberfläche des Patienten.

Figur 2 zeig die Konstruktion der Ableiterelektrode 10 in einem teilmontierten Zustand. Die Kontaktlasche 12 ist zwischen den beiden Scheiben 13 angeordnet, die Dommutter 14 ist durch eine Durchtrittsöffnung 23 in dem Prothesenschaft 20 hindurchgeführt. Die Abdeckkappe 11 ist bereits auf dem Kopf der Dommutter 14 aufgeklipst. Die Dommutter 14 weist ein Innengewinde 141 auf, das in einem Schaft 140 der Dommutter 14 angeordnet ist. Der Schaft 140 erstreckt sich von dem Kopf der Dommutter 14 in Richtung auf die Innenseite 22 des Prothesenschaftes 20. Darüber hinaus weist der Schaft 140 eine umlaufende Nut 142 auf der Außenseite auf, in der ein Dichtelement angeordnet werden kann, beispielsweise ein geschlossener Dichtring. Der Außendurchmesser des Schaftes 140 entspricht im Wesentlichen dem Innendurchmesser der Durchtrittsöffnung 23. Die Länge des Schaftes 140 ist in dem dargestellten Ausführungsbeispiel so bemessen, dass er im Wesentlichen bündig mit der Oberfläche auf der Innenseite 22 des Prothesenschaftes 20 abschließt. Grundsätzlich können auch kürzere oder längere Schäfte 140 vorgesehen sein.

Auf der Innenseite 22 ist die Domschraube 15 mit einem Bolzen 150 angeordnet. Der Bolzen 150 weist ein Außengewinde 151, das mit dem Innengewinde 141 der Dommutter 14 korrespondiert. Das Außengewinde 151 ist nur teilweise in das Innengewinde 141 eingeschraubt. Der Bolzen 150 mündet in eine Domkappe oder dem sogenannten Dom 152, der leicht gewölbt oder kalottenförmig oder pilzartig ausgebildet ist. Von dem Außenrand des Domes 152 erstreckt sich die Rückseite, die der Innenseite 22 des Prothesenschaftes 20 zugewandt ist, leicht nach innen, so dass bevorzugt nur ein Auflagering oder eine Auflageringfläche vorhanden ist. Sowohl der Dom 152 als auch der Bolzen 150 und die Dommutter 14 sind elektrisch leitend ausgebildet. Erregungspotentiale durch Muskelaktivitäten werden über den Dom 152 und die Dommutter 14 über die Kontaktlasche 12, die elektrisch leitend mit der Dommuter 14 gekoppelt ist, zu einem nicht dargestellten Kabel geleitet, das dann mit einem Stecker verbunden ist. Über das Kabel und den Stecker werden die detektierten elektrischen Potentiale von Muskelaktivitäten beispielsweise zur Auswertung oder zur Steuerung einer Prothesenkomponente weitergeleitet.

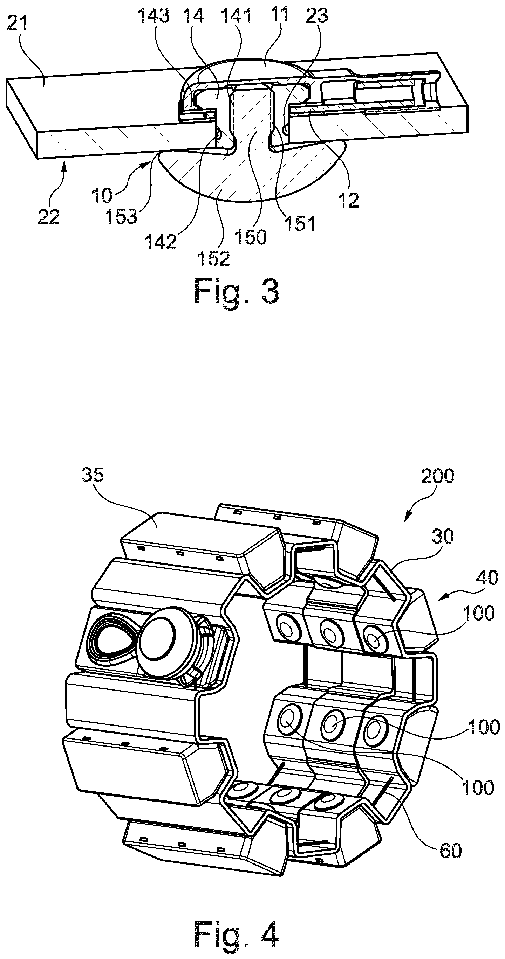

Figur 3 zeigt den montierten Zustand in Schnittdarstellung. Die Domschraube 15 ist vollständig mit der Dommutter 14 verschraubt. Der Dom 152 liegt mit seinem unterseitigen Rand auf der Innenseite 22 des Prothesenschaftes 20 auf und wird durch die Pressung feuchtigkeitsdicht gegen den Prothesenschaft 20 gedrückt. Eine weitere Abdichtung der Durchtrittsöffnung 23 erfolgt durch den Dichtring in der Außennut 142 sowie die beiden Scheiben 13, die die Kontaktlasche 12 einklemmen.

Die Montage der Ableiterelektrode 10 erfolgt nach der Bohrung oder anderweitigen Erzeugung der Durchtrittsöffnung 23 in dem Prothesenschaft 20. Vorteilhafterweise wird zunächst der Bolzen 150 mit dem Außengewinde 151 der Domschraube 15 durch die Durchtrittsöffnung 23 gesteckt und in der Position gehalten. Vormontiert ist die Dommutter 14 mit den aufgesteckten Scheiben 13, der dazwischen angeordneten Kontaktlasche 12 sowie gegebenenfalls einem Dichtring. Die Abdeckkappe 11 ist noch nicht über der Dommutter 14 angeordnet. In der Dommutter 14 kann beispielsweise eine Innensechskantausnehmung oder ein Schlitz angeordnet sein, um mit nur einen Werkzeug eine feuchtigkeitsdichte, elektrisch durchleitende Befestigung der Ableiterelektrode 10 an der Wand des Prothesenschaftes 20 durchzuführen. Abschließend wird die Abdeckkappe 14 aufgeklipst und durch einen Hinterschnitt, der in der Dommutter 14 ausgebildet ist, formschlüssig fixiert. Das Kabel wird durch die Umhüllung der Kabelaufnahme der Kontaktlasche 12 hindurchgesteckt, sodass eine elektrische Isolierung nach außen hin einfach und schnell erzielt wird. Mit einer solchen Elektrode ist eine geringere Aufbauhöhe über dem Schaft, insbesondere auf der Außenseite 21 möglich. Eine zügige Montage der Elektrode mit dem Dom 152 ist möglich. Über eine Schraubverbindung ist eine reversible Montage möglich, gegen ein unbeabsichtigtes Lösen der Verbindung ist die Dommutter 14 über den Dichtring sowie die beiden Scheiben 13 gesichert. Die Scheiben 13 können als Quetschscheiben ausgebildet sein. Die Dommutter 14 kann mit der elektrischen Kontaktierung und dem Dichtring vormontiert werden, die Scheiben 13 und die Kontaktlasche 12 werden über den Dichtring gegen ein unbeabsichtigtes Herunterrutschen gesichert. Über die Verschraubung ist eine Anpassung an unterschiedliche Wandstärken des Prothesenschaftes 20 möglich.

Die obigen Ausführungen gelten sowohl für Ableiterelektroden 10, die an einem Prothesenschaft 20 befestigt werden, als auch für Oberflächenelektroden 100, die an einer Oberflächenelektrodenanordnung 200, beispielsweise einer Manschette oder dergleichen, montiert werden, was nachfolgend erläutert wird.

In der Figur 4 ist eine Oberflächenelektrodenanordnung 200 gezeigt, bei der die Oberflächenelektroden 100 analog zu den Ableiterelektroden 10 der Figuren 1 bis 3 aufgebaut sind. Die Oberflächenelektrodenanordnung 200 ist als Manschette ausgebildet und weist ein Trägerelement auf, das wellenförmig ausgebildet ist. Die Oberflächenelektroden 100 sind auf den nach innen gerichteten wellenförmigen Vorsprüngen des Trägerelementes 30 angeordnet, um einen sicheren Hautkontakt gewährleisten zu können. Die linienförmig angeordneten Oberflächenelektroden 100, die dreipolig aufgebaut sind, sind gleichmäßig oder zumindest annähernd gleichmäßig über den Umfang des als geschlossener Ring ausgebildeten Trägerelementes 30 angeordnet. Es können auch leichte Abweichungen in dem Abstand der Elektroden oder Elektrodenlinien über den Umfang vorgenommen werden. Auf der Außenseite des Trägerelementes 30 ist den Oberflächenelektroden 100 jeweils eine Verstärkereinrichtung 40 zugeordnet, in der die aufgenommenen myoelektrischen Signale verstärkt und gegebenenfalls vorverarbeitet und weitergeleitet werden. Jede Verstärkereinrichtung 40 ist in einem länglichen Gehäuse 35 angeordnet und kann einen eigenen Energiespeicher aufweisen. Ein Versorgungsanschluss 50 ist in einer nicht belegten Vertiefung auf der Außenseite des Trägerelementes 30 angeordnet, über den den einzelnen Verstärkereinrichtungen 40 Energie zugeführt werden kann. Darüber hinaus kann über den Versorgungsanschluss 50 eine gemeinsame Ableitung der Oberflächenelektroden Signale zu einer Steuerungseinrichtung erfolgen. Ebenfalls ist eine Spanneinrichtung 42 vorgesehen, über die der Umfang der Manschette oder des Trägerelementes 30 einstellbar ist

Aufgrund der gewellten Ausführungsform des Trägerelementes 30 ist es möglich, einen elastischen und radial aufweitbaren Aufbau zu erreichen, sodass die Oberflächenelektrodenanordnung 200 an Gliedmaßen mit unterschiedlichen Durchmessern und Umfängen angelegt werden können. Dadurch ist es möglich, die Oberflächenelektrodenanordnung 200 im Rahmen einer Erstversorgung und Erstevaluation von Patienten einzusetzen, ohne dass es eine Maßanfertigung eines Prothesenschaftes bedarf.

In dem dargestellten Ausführungsbeispiel sind an dem Trägerelement 30 acht dreipolige Oberflächenelektrodentriplets angeordnet. Das Trägerelement 30 und damit auch die Oberflächenelektrodenanordnung 200 ist, wie bereits ausgeführt, in Umfangsrichtung elastisch ausgebildet, in Axialerstreckung, also in proximal-distal Richtung oder in Längserstreckung der linienförmig hintereinander angeordneten Oberflächenelektroden 100 eines Elektrodentriplets längenunveränderlich oder steif angeordnet. Dadurch ist es möglich, einen festen Abstand zwischen den Oberflächenelektroden 100 eines Elektrodentriplets oder bei zwei Elektroden eines Elektrodenpaares zu definieren. Die Oberflächenelektrodenanordnung 200 ist an einem Gliedmaßenstumpf verdrehbar und verschieblich angeordnet, um die optimale Position der Oberflächenelektroden 100 zur Ableitung eindeutiger myoelektrischer Signale bestimmen zu können.

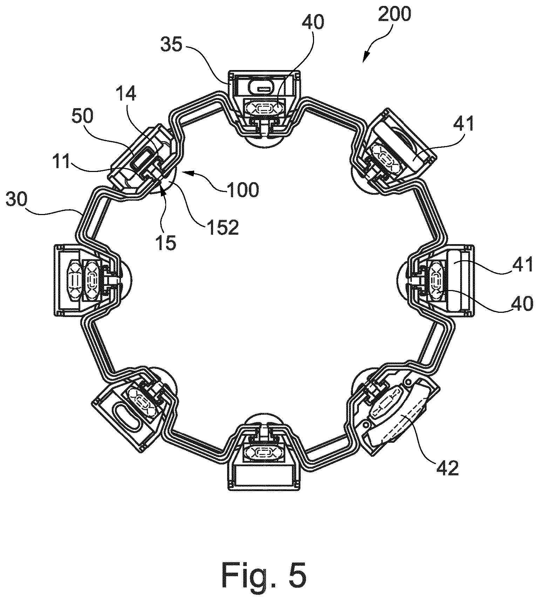

Figur 5 zeigt die Oberflächenelektrodenanordnung 200 gemäß Figur 4 in Querschnittsansicht. Die einzelnen Oberflächenelektroden 100 sind analog zu den Figuren 1 bis 3 als Domschrauben 15 mit Dommuttern 14 aufgebaut. Die insgesamt acht Elektrodenreihen sind paarweise aneinander gegenüberliegend um den Umfang verteilt angeordnet. In der Schnittdarstellung ist zu erkennen, dass in den Verstärkereinrichtungen 40 nur teilweise Energiespeicher 41 angeordnet sind. Zwischen den einzelnen Elektroden 100 kann ein elastisches Seil oder ein Band 60 oder auch ein zugstarres, flexibles Seil mit des Spanneinrichtung 42 angeordnet sein, um die Kompressionswirkung zu erhöhen und eine Rückstellung nach einem Aufweiten zu erleichtern und zu ermöglichen.

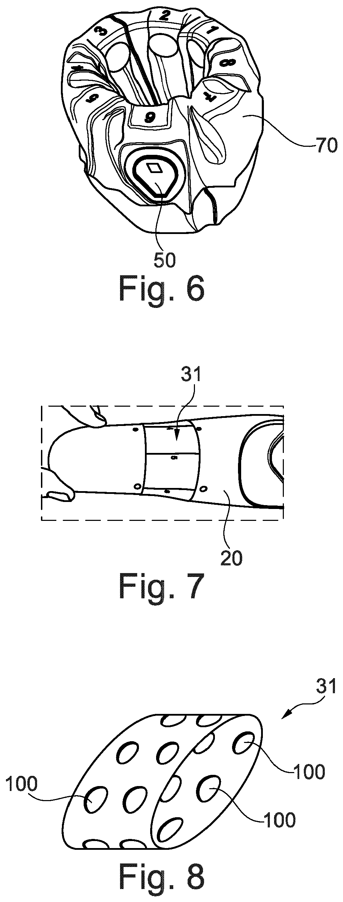

Figur 6 zeigt eine perspektivische Ansicht einer Oberflächenelektrodenanordnung 200 mit einem darum angeordneten und darüber gezogenen Überzug 70, der aus einem hochflexiblen Gewebe bestehen kann. Der Überzug 70 ist sowohl auf der Innenseite als auch auf der Außenseite der Oberflächenelektrodenanordnung 200 angeordnet und umgibt diese nahezu vollständig. Lediglich die Dome 152 der Oberflächenelektroden 100 auf der Innenseite des ringförmigen oder hülsenförmigen Trägerelementes 30 sind frei gelassen, um einen direkten Hautkontakt zu ermöglichen.

Darüber hinaus ist auf der Außenseite der Oberflächenelektrodenanordnung 200 der Versorgungsanschluss 50 frei gelassen, um Energie und Daten von den Verstärkereinrichtungen 40 beziehungsweise den Oberflächenelektroden 100 zu leiten. Der Überzug 70 ist lösbar an dem Trägerelement 30 befestigt. An den Domen 152 ist über die leicht gerundete Ausformung der pilzförmigen Köpfe ein Hinterschnitt 153 gebildet, sodass sich das Gewebe mit dem entsprechenden Loch oder mit der entsprechenden Ausnehmung 71 in den Zwischenraum oder Hinterschnitt legen kann. Die Festlegung auf der Außenseite kann über einen Klettverschluss oder über Knöpfe oder Druckknöpfe erfolgen, sodass der Überzug 70 leicht anlegbar und wieder entfernbar ist.

An der einen Stirnseite der Oberflächenelektrodenanordnung 200 sind Markierungen in Gestalt von Ziffern aufgebracht, um eine eindeutige Zuordnung der Elektroden oder Elektrodenpaare über den Umfang zu erreichen. Dadurch wird die Signalauswertung erleichtert und das jeweilige Signal kann einer Reihe von Oberflächenelektroden 100 beziehungsweise einem Oberflächenelektrodenpaar zugeordnet werden.

Eine Variante der Erfindung in der Figur 7 zeigt, wie eine einmal gefundene Position der Oberflächenelektroden 100 einer Oberflächenelektrodenanordnung 200 auf einem Gliedmaßenstumpf 1 auf einen Prothesenschaft 20 übertragen werden kann. Dies geschieht in dem Ausführungsbeispiel über eine Positionierhilfe 31, die aus einer elastischen Textilmanschette besteht, auf deren Außenseite am Umfang gleichmäßig beabstandete Markierungen angeordnet sind, z.B. in Gestalt fortlaufender Ziffern von 1 bis 8. Die Markierungen verändern ihren Abstand zueinander bevorzugt gleichmäßig, wenn sich das Textil ausdehnt, um sich an den jeweiligen Umfang der Gliedmaße anzupassen. In einer Ausführungsform weist das Textil in Umfangsrichtung ein gleichmäßiges Dehnungsverhalten auf. Die Breite der Manschette oder des Bandes gibt dabei den optimalen Abstand der Elektroden in Axialerstreckung vor, also in proximal-distal- Richtung oder entlang der Längserstreckung des Gliedma-ßenstumpfes, die Markierungen geben die Abstände der Elektroden in Umfangsrichtung an. In der Figur 7 ist die Positionierhilfe an einem Prothesenschaft 20 angelegt. Über die Markierungen können die jeweiligen Positionen der Elektroden und damit der Stellen für eine Durchtrittsöffnung auf dem Prothesenschaft 20 angezeichnet werden. Statt der Markierungen können auch entsprechend beabstandete Löcher in der Positionierhilfe ausgebildet sein, die als Schablone für die optimalen Positionen der Durchtrittsöffnungen und damit der Elektroden dienen. Eine solche Variante ist in der Figur 8 gezeigt.

Die an dem Trägerelement befestigten Oberflächenelektroden 100 sowie die an dem Prothesenschaft 20 zu positionierenden Ableiterelektroden 10 sind annähernd äquidistant über den Umfang verteilt und in Längserstreckung des Gliedmaßenstumpfes zueinander beabstandet. Um eine Verkürzung des vorgesehenen Elektrodenabstandes zueinander zu verringern, können in Längsrichtung der Positionierhilfe 31 Stabilisierungselemente angeordnet, sodass die Positionierhilfe 31 zwar radial aufgeweitet, jedoch axial nicht verkürzt wird. Das Trägerelement einer Oberflächenelektrodenanordnung 200 kann auch aus einem elastischen Textil bestehen oder ein solches aufweisen und mit Stabilisierungselementen ausgestattet sein.

Figur 8 zeigt eine Variante einer Positionierhilfe 31 mit Markierungen und/oder Löchern oder einer Oberflächenanordnung 200 mit Oberflächenelektroden 100. Die Oberflächenelektrodenanordnung 200 oder Positionierhilfe 31 ist bandförmig ausgebildet und nicht als ein geschlossener Ring. Die Oberflächenelektrodenanordnung 200 oder Positionierhilfe 31 kann um eine Gliedmaße herum angelegt und dort befestigt werden. Der Durchmesser oder der Umfang ist veränderlich ausgebildet, beispielsweise dadurch, dass an der Außenseite ein Klettverschluss angeordnet ist, sodass eine Oberflächenelektrodenanordnung 200 an Gliedmaßenstümpfen und Positionierhilfen 31 an Prothesenschäften 20 mit unterschiedlichem Umfang oder Durchmesser angeordnet werden können.

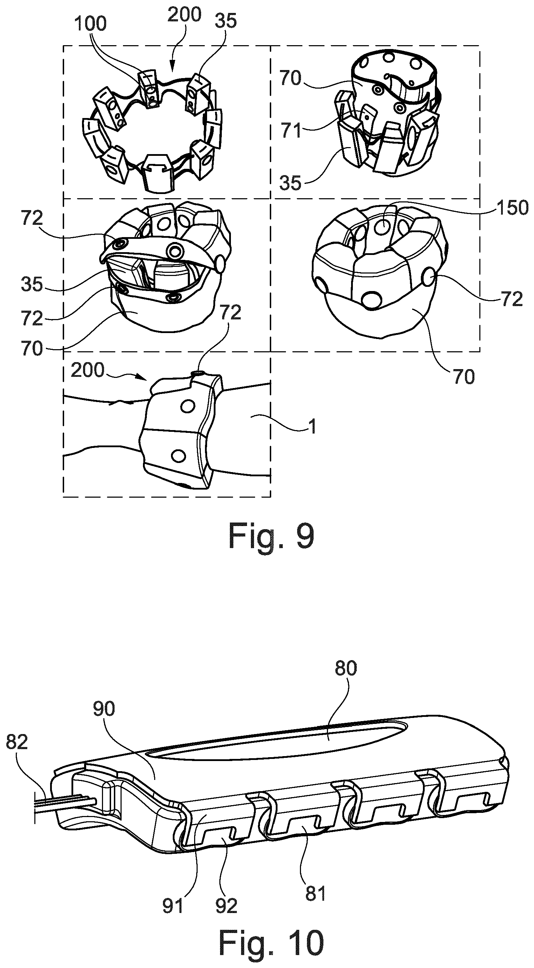

Figur 9 zeigt den Ablauf des Anlegens einer Oberflächenelektrodenanordnung 200 an einen Patienten. In der Darstellung links oben ist die Oberflächenanordnung einer perspektivischen Ansicht gezeigt. Die Verstärkereinrichtungen 40 sind in den lang gestreckten Gehäusen 35 angeordnet, die gleichzeitig Stabilisierungselemente ausbilden. Innerhalb der Gehäuse 35 sind die Oberflächenelektroden 100 in Längserstreckung der Gehäuse 35 zueinander beabstandet angeordnet. Die einzelnen Verstärkereinrichtungen 40 in den jeweiligen Gehäusen 35 sind über Kabel miteinander verbunden, ein Seil 60 kann über eine Spanneinrichtung 42 gespannt oder entspannt werden, um die ringartig angeordneten Oberflächenelektroden 100 beziehungsweise Gehäuse 35 der Oberflächenelektrodenanordnung 200 hinsichtlich ihres maximalen Umfangs einstellen zu können.

In dem zweiten Bild oben rechts wird der Überzug 70 in die kreisförmige Anordnung der Oberflächenelektrodenanordnung eingesetzt. Der Überzug 70 ist als Schlauch oder Hülse ausgebildet und weist korrespondierend zu den Domen 152 ausgebildete Ausnehmungen 71 auf, durch die die Dome 152 hindurchgeführt werden. An den proximalen und distalen Stirnseiten, also in Längserstreckung an den Endseiten der Manschette 30 stehen Abschnitte des Überzuges 70 über. Wenn alle Dome 152 durch die Ausnehmungen 71 hindurchgeführt worden sind, werden die in Längserstreckung überstehenden Bereiche des Überzuges 70 umgeschlagen, sodass sie auf der Außenseite der Gehäuse 35 zur Anlage kommen. Dieser Schritt ist in der mittleren, linken Darstellung der Figur 9 gezeigt. An dem Überzug 70 sind Verschlusselemente 72 angeordnet, die in dem dargestellten Ausführungsbeispiel als Druckknöpfe ausgebildet sind. Alternative Verschlusselemente 72 können vorgesehen sein, beispielsweise Klettverschlüsse oder Knöpfe. Die Verschlusselemente 72 werden miteinander in Eingriff gebracht, sodass die Außenseite der Oberflächenelektrodenanordnung 200 vollständig von dem Überzug 70 umgeben ist. Der vollständig montierte Zustand des Überzuges 70 an der Oberflächenelektrodenanordnung 200 ist in der unteren rechten Darstellung gezeigt. Die Verschlusselemente 72 liegen auf der Außenseite der Oberflächenelektrodenanordnung, die Dome 152 sind an der Innenseite des hülsenartigen Trägerelementes 30 ausgebildet.

Die linke untere Darstellung der Figur 9 zeigt die Oberflächenelektrodenanordnung 200 mit Überzug 70 im angelegten Zustand. Der Überzug 70 kann elastische Rückstellkräfte bereitstellen, ebenso ist es möglich, dass der Umfang über die Spanneinrichtung 42 an einem der Gehäuse 35 der Oberflächenelektrodenanordnung eingestellt wird.

Figur 10 zeigt eine perspektivische Darstellung einer Steuerungseinheit 80 mit einem Gehäuse, an dessen Außenseite Steckeraufnahmen 81 ausgebildet sind, in die nicht dargestellte Stecker, die über Kabel mit den Oberflächenelektroden 100 oder den Ableiterelektroden 10 verbunden sind, verbunden werden können. Die Steckeraufnahmen 81 oder Buchsen sind in dem dargestellten Ausführungsbeispiel aneinander gegenüberliegenden Längsseiten eines flachen, leicht gebogenen Gehäuses angeordnet. In dem dargestellten Ausführungsbeispiel sind acht Steckeraufnahmen 81 insgesamt vorgesehen, vier an jeder Längsseite. Die leichte Krümmung ist vorgesehen, um die Steuerungseinheit 80 leichter an einem Prothesenschaft 20 oder an einem Patienten festlegen zu können. Die Steuerungseinheit 80 weist einen Kabelausgang 82 auf, über den die Steuerungseinheit 80 mit einer nicht dargestellten, angetriebenen prothetischen Komponente 2 gekoppelt ist, um Steuerungssignale auf der Grundlage eines ausgewählten Steuerungsverfahrens den jeweiligen Antrieb zu übermitteln. Basierend auf den von den Oberflächenelektroden 100 oder den Ableiterelektroden 10 empfangenen myoelektrischen Signalen werden die jeweiligen Antriebe angesteuert und aktiviert beziehungsweise deaktiviert. Die Aktivierung erfolgt sowohl hinsichtlich Verstellgeschwindigkeit als auch Verstellweg als auch Verstelldauer und/oder der Stärke.

Der Steuerungseinheit 80 zugeordnet ist ein Sicherungselement 90, dass an der nach außen gekrümmten Außenseite des Gehäuses der Steuerungseinheit 80 klemmend festgelegt ist. Das Sicherungselement 90 weist im Bereich der Steckeraufnahmen 81 Klammern oder Laschen 91 auf, die die Öffnung der Steckeraufnahme 81 teilweise überdecken. In den Klammern oder Laschen 91 ist jeweils eine Ausnehmung 92 ausgebildet, die zur Durchführung des Kabels dient. Zur Festlegung der Steckerverbindung von den Oberflächenelektroden 100 oder Ableiterelektroden 10 zu der Steuerungseinheit 80 werden die Stecker 19 an den Kabeln 18 von Elektroden 10, 100, in die Steckeraufnahmen 81 eingeführt. Wenn alle Stecker in die Steckeraufnahmen 81 eingeführt sind, wird in das Sicherungselement 90 über das Gehäuse der Steuerungseinheit 80 gelegt und klemmend festgehalten. Die Laschen oder Klammern 91 verhindern ein Herausziehen der Stecker entgegen der Einführrichtung, durch die Ausnehmungen 92 in den Laschen oder Klammern 91 bleiben die Kabel jedoch im Wesentlichen geradlinig in Einführrichtung herausführbar, sodass keine Knickstellen entstehen. Das Sicherungselement 90 stellt eine lösbare, mechanische Sicherung gegen ein unbeabsichtigtes Entkontaktieren der Stecker bereit.

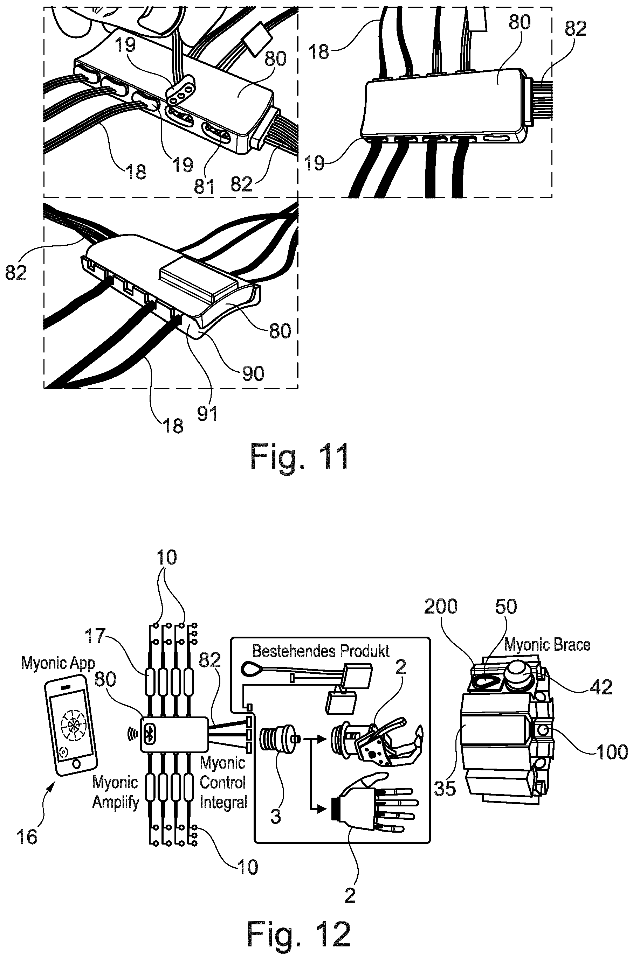

Figur 11 zeigt eine Variante der Erfindung mit der Steuerungseinheit 80 und einem Gehäuse, das korrespondierend zu der Figur 10 an den schmalen Längsseiten des Gehäuses insgesamt zehn Steckeraufnahmen 81 aufweist, fünf auf jeder Seite. In dem linken oberen Bild der Figur 11 sind bereits einige Stecker 19 in die Steckeraufnahmen 81 eingeführt. Die Stecker 19 sind als dreipolige Stecker ausgeführt, an denen die Kabel 18 von den einzelnen Elektroden 10, 100 befestigt sind. In der linken oberen Darstellung der Figur 11 sind bereits sieben Stecker 19 in die Steckeraufnahmen 81 der Steuerungseinheit 80 eingeführt. In der oberen rechten Darstellung sind alle Steckerverbindungen der Oberflächenelektroden 100 der Manschette gemäß Figur 4 eingeführt. Die untere linke Darstellung zeigt die gesicherte Darstellung mit acht eingeführten Steckern 19, davon im Wesentlichen geradlinig abgehende Kabel 18 und dem Sicherungselement 90 mit den Klammern 91 und den Ausnehmungen 92. Anders als in der Figur 10 ist in dem Ausführungsbeispiel gemäß Figur 11 das Sicherungselement 90 auf der Innenseite des Gehäuses der Steuerungseinheit 80 angeordnet. Der Kabelausgang 82 verbindet die Steuerungseinheit 80 mit der zu steuernden Prothesenkomponente.

Figur 12 zeigt eine schematische Darstellung des Prothesensystems mit einer Prothesenkomponente 2, die in Gestalt eines bestehenden Produktes ausgebildet ist. An einem Träger 3 ist die Prothesenkomponente 2 festlegbar. Im dargestellten Ausführungsbeispiel sind zwei unterschiedliche Prothesenkomponenten 2 zur Auswahl möglich, einmal eine einfache Greifeinrichtung, die über eine Zweikanalsteuerung angesteuert werden kann. Die Greifeinrichtung kann greifen und lösen, sodass ein einzelner Antrieb aktiviert, reversiert und angehalten werden muss, um die gewünschte Tätigkeit ausführen zu können. Die untere Prothesenkomponente 2 ist eine Prothesenhand mit einer Vielzahl angetriebener Prothesenfinger, die unterschiedliche Antriebe aufweisen, sodass eine wesentlich komplexere Steuerung vorliegen muss, um mit von dem Patienten abgeleiteten Signalen, insbesondere elektrischen Signalen, eine Steuerung vornehmen zu können. Der Träger 3 ist über den Kabelausgang 82 mit der Steuerungseinheit 80 gekoppelt. Die Steuerungseinheit 80 ist über Stecker 19 und Kabel 18 mit den Ableiterelektroden 10 verbunden. Die Elektrodenpaare sind dargestellt, ebenso ein Elektrodentriplett mit zusätzlichen Ableiterelektroden als Masse.

Zwischen den Ableiterelektroden 10 und der Steuerungseinheit 80 kann ein Adapter 17 angeordnet sein, über den Signale der Ableiterelektroden 10, die eigentlich für eine Steuerung auf Basis einer Mustererkennung geeignet und vorgesehen sind, in Zweikanalsteuerungssignale umwandelt werden, die für die Steuerung der Greifeinrichtung auf Basis einer Zweikanalsteuerung verwendet werden können. Die Steuerungseinheit 80 kann drahtlos, beispielsweise über Bluetooth, mit einem Bediengerät 16, beispielsweise einem Computer, einem Tablet oder einem Smartphone, gekoppelt werden, um die Steuerung einzurichten. Das Bediengerät 16 kann eine Authentifizierungssoftware enthalten, über die festgelegt wird, dass nur eine autorisierte Person die Steuerung, die der Betätigung der jeweiligen Prothesenkomponente 2 zugrunde gelegt wird, verändern kann.

Weiterhin ist als weitere Prothesensystemkomponente die Oberflächenelektrodenanordnung 200 gezeigt, die wie oben beschrieben ausgebildet ist und insbesondere verschieblich und verdrehbar um einen Gliedmaßenstumpf 1 herum angelegt werden kann. Die Oberflächenelektroden 100 auf der Innenseite der Oberflächenelektrodenanordnung 200 an dem Trägerelement 30 können entweder per Kabel 18 und Stecker 19 mit einer Steuerungseinheit 80 gekoppelt werden, um bereits ohne einen fertigen Prothesenschaft 2 möglichst schnell Informationen darüber zu erhalten, welche elektrischen Signale von einem Patienten erzeugt werden können und welche nicht. Auf Basis dieser von der Oberflächenelektrodenanordnung 200 aufgenommenen elektrischen Signale wird entweder in der Steuerungseinheit 80 oder in einer anderen Auswertungseinrichtung oder einem Controller entschieden, ob eine Steuerung über eine Musterkennungssoftware oder eine Zweikanalsteuerung günstig und geeignet ist. Die Oberflächenelektrodenanordnung 200 kann in eine optimierte Position gebracht werden, die für die Ableitung der myoelektrischen Signale am günstigsten ist. Diese Position und die Zuordnung der jeweiligen Elektroden 100 oder der Elektrodenpaare, beispielsweise in dem jeweiligen Gehäuse 35, werden gespeichert. Über die Markierungen auf der Stirnseite oder an dem Umfang des Trägerelementes 30 oder der Oberflächenelektrodenanordnung 200 wird festgelegt, welche Elektrode 10 an welcher Stelle zu liegen hat oder angeordnet werden soll. Diese Positionierung der jeweiligen Elektroden 10 oder Elektrodenpaare wird dann auf einen endgültigen Prothesenschaft 20 übertragen. Die Übertragung kann entweder über eine Projizierung, die Anfertigung einer Schablone, eine Positionierhilfe 31 oder das Überlagern der gewählten Position auf einer Abbildung erfolgen, sodass der Orthopädietechniker bei der Herstellung des Prothesenschaftes 20 weiß, wo welche Durchtrittsöffnung 23 zur Befestigung und Positionierung der Ableiterelektrode 10 angeordnet werden soll.

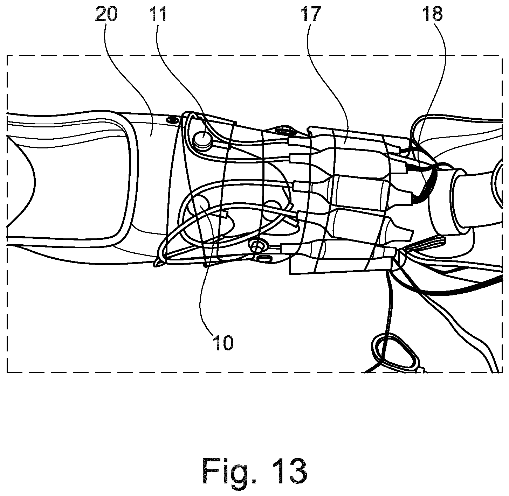

In der Figur 13 ist die endgültige Anordnung der Ableiterelektroden 10 mit den Abdeckkappen 11 an dem Prothesenschaft 2 gezeigt. Die Ableiterelektroden 10 sind über die Kabel 18 und einem Adapter 17 mit der nicht dargestellten Steuerungseinheit 80 gekoppelt, die dann wiederum die nicht dargestellte Prothesenkomponente 2, die an dem distalen Ende des Prothesenschaftes 20 befestigt ist, mit den entsprechenden Befehlen für den jeweiligen Antrieb versorgt.

### Bezugszeichenliste

- 1: Gliedmaßenstumpf
- 2: Prothesenkomponente
- 3: Träger
- 10: Ableiterelektrode
- 11: Abdeckkappe
- 12: Kontaktlasche
- 13: Scheibe
- 14: Dommutter
- 15: Domschraube
- 16: Bediengerät
- 17: Adapter
- 18: Kabel
- 19: Stecker
- 20: Prothesenschaft
- 21: Außenseite
- 22: Innenseite
- 23: Durchtrittsöffnung
- 30: Trägerelement
- 31: Positionierhilfe
- 35: Gehäuse
- 40: Verstärkereinrichtung
- 41: Energiespeicher
- 42: Spanneinrichtung
- 50: Versorgungsanschluss
- 60: Seil
- 70: Überzug
- 71: Ausnehmung
- 72: Verschlusselement
- 80: Steuerungseinheit
- 81: Steckeraufnahme
- 82: Kabelausgang
- 90: Sicherungselement
- 91: Klammer
- 92: Ausnehmung
- 100: Oberflächenelektrode
- 140: Schaft
- 141: Innengewinde
- 142: Nut
- 143: Hinterschnitt
- 150: Bolzen
- 151: Außengewinde
- 152: Dom
- 153: Hinterschnitt
- 200: Oberflächenelektrodenanordnung

## Patentansprüche

1. Verfahren zum Einrichten eines myoelektrisch gesteuerten Prothesensystems mit einem Prothesenschaft (20) und mehreren Ableiterelektroden (10) zur Erfassung elektrischer Muskelaktivitäten mit den Schritten:
- Anlegen einer Oberflächenelektrodenanordnung (200) mit mehreren Oberflächenelektroden (100) um den Umfang eines Gliedmaßenstumpfes (1)
- Erfassen von elektrischer Muskelaktivität an Muskeln des Gliedmaßenstumpfes (1) als myoelektrische Signale durch die Oberflächenelektroden (100)
- Auswerten der myoelektrischen Signale hinsichtlich ihrer Signalqualität
- Auswahl des Steuerungsverfahrens, mit dem das Prothesensystem gesteuert wird, auf der Grundlage der Auswertung der Signalqualität, und
- Festlegen der Ableitelektroden (10) an dem Prothesenschaft (20).

2. Verfahren nach Anspruch 1, wobei die Oberflächenelektrodenanordnung (200) unabhängig von dem Prothesenschaft (20) ausgebildet ist und separat von dem Prothesenschaft (20) angelegt wird.

3. Verfahren nach Anspruch 1, oder 2, wobei die Oberflächenelektrodenanordnung (200) verschieblich und/oder verdrehbar an dem Gliedmaßenstumpf (1) angelegt wird.

4. Verfahren nach einem der voranstehenden Ansprüche, wobei die Oberflächenelektroden (100) gleichmäßig oder im Wesentlichen gleichmäßig zueinander beabstandet um den Gliedmaßenstumpf (1) angeordnet werden.

5. Verfahren nach einem der voranstehenden Ansprüche, wobei die Signalqualität der myoelektrischen Signale der Oberflächenelektroden (100) auf der Grundlage ihrer Amplitude, Dauer, Frequenz und/oder Brandbreite bewertet wird.

6. Verfahren nach einem der voranstehenden Ansprüche, wobei eine Auswahl und Festlegung einer Grundeinstellung des Steuerungsverfahrens vorgenommen wird.

7. Verfahren nach Anspruch 6, wobei die Auswahl des Steuerungsverfahrens automatisch in einer Steuerungseinheit (80) auf der Grundlage definierter Kriterien getroffen wird.

8. Verfahren nach Anspruch 6 oder 7, wobei als Steuerungsverfahren eine Mustererkennung ausgewählt und eine ausschließlich von einer autorisierten Person modifizierbare und löschbare Grundeinstellung des Mustererkennungsverfahrens vorgenommen wird.

9. Verfahren nach Anspruch 6 oder 7, wobei als Steuerungsverfahren eine Zweikanalsteuerung ausgewählt wird und die beiden am besten unterscheidbaren myoelektrischen Signale identifiziert und die Position der dazugehörigen Elektroden (10, 100) an dem Stumpf (1) in einer Anzeigevorrichtung angezeigt werden.

10. Verfahren nach einem der voranstehenden Ansprüche, wobei die Position der Oberflächenelektroden (100) durch eine Positionierhilfe (31) von dem Gliedmaßenstumpf (1) auf den Prothesenschaft (20) übertragen wird.

11. Verfahren nach einem der voranstehenden Ansprüche, wobei die Oberflächenelektrodenanordnung (200) auf dem Gliedmaßenstumpf (1) in eine Endstellung, in der zumindest zwei Signale detektierbar sind, verschoben und/oder verdreht wird und die Ableiterelektroden (10) korrespondierend zu der Endstellung an dem Prothesenschaft (20) festgelegt werden.

12. Verfahren nach Anspruch 10, wobei die Position der Ableiterelektroden (10) an dem Prothesenschaft (20) angezeigt, auf dem Prothesenschaft (20) projiziert oder über eine Positionierungslehre auf den Prothesenschaft (20) übertragen wird.

13. Verfahren nach einem der voranstehenden Ansprüche, wobei die Ableiterelektroden (10) über eine Domverschraubung an dem Prothesenschaft (20) festgelegt werden.

14. Verfahren nach einem der voranstehenden Ansprüche, wobei die Ableiterelektroden (10) über Kabel (18) mit einer Steuerungseinheit (80) verbunden werden und mechanisch über ein Sicherungselement (90) gegen ein Lösen gesichert werden.

15. Prothesensystem mit einem Prothesenschaft (20), der an einem Gliedmaßenstumpf (1) anlegbar ist, und mehreren Ableiterelektroden (10) zur Erfassung elektrischer Muskelaktivitäten, einer mit den Ableiterelektroden (10) verbunden Steuerungseinheit und einer angetriebenen Prothesenkomponente (2), die an dem Prothesenschaft (20) befestigt und mit der Steuerungseinheit (80) gekoppelt ist, mit
einer Oberflächenelektrodenanordnung (200) mit mehreren Oberflächenelektroden (100), die um den Umfang eines Gliedmaßenstumpfes (1) anlegbar sind, wobei die Oberflächenelektrodenanordnung (200) verschieblich und/oder verdrehbar an dem Gliedmaßenstumpf (1) anordenbar ist,
**dadurch gekennzeichnet, dass** die Steuerungseinheit (80) über Stecker (19) mit den Ableiterelektroden (10) oder Oberflächenelektroden (100) lösbar verbunden ist und dass zwischen der Steuerungseinheit (80) und dem Stecker (19) ein Adapter (17) zur Umwandlung myoelektrischer Signale, deren Qualität und Unterscheidbarkeit für eine Mehrkanalsteuerung geeignet sind, in Zweikanalsteuerungssignale angeordnet ist.

16. Prothesensystem nach Anspruch 15, wobei die Oberflächenelektrodenanordnung (200) als geschlossenes, ringförmiges Band ausgebildet ist.

17. Prothesensystem nach Anspruch 15 oder 16, wobei
die Oberflächenelektrodenanordnung (200) in Umfangsrichtung elastisch ausgebildet ist.

18. Prothesensystem nach einem der Ansprüche 15 bis 17, wobei
die Oberflächenelektroden (100) in Umfangsrichtung gleichmäßig oder im Wesentlichen gleichmäßig zueinander beabstandet an der Oberflächenelektrodenanordnung (200) angeordnet sind.

19. Prothesensystem nach einem der Ansprüche 15 bis 18, wobei die Oberflächenelektrodenanordnung (200) eine Positionierhilfe (31) und/oder zumindest ein sich in proximal-distal-Richtung erstreckendes Stabilisierungselement (35) aufweist.

20. Prothesensystem nach einem der Ansprüche 15 bis 19, wobei an der Oberflächenelektrodenanordnung (200) ein auswechselbarer Überzug (70) angeordnet ist.

21. Prothesensystem nach Anspruch 20, wobei an einer Positionierhilfe (31) und/oder dem Überzug (70) Markierungen angeordnet sind.

22. Prothesensystem nach Anspruch 20 oder 21, wobei in dem Überzug (70) Ausnehmungen (71) für Oberflächenelektroden (100) ausgebildet sind.

23. Prothesensystem nach einem der Ansprüche 15 bis 22, wobei die Oberflächenelektroden (100) und/oder Ableiterelektroden (10) als Domschraube (15) mit Dommutter (14) ausgebildet und mit der Oberflächenelektrodenanordnung (200) oder dem Prothesenschaft (20) verschraubt sind.

24. Prothesensystem nach Anspruch 23, wobei die Domschraube (15) einen Hinterschnitt (153) aufweist und die Durchtrittsstelle (23) gegenüber Feuchtigkeitsdurchtritt abgedichtet ist.

25. Prothesensystem nach einem der Ansprüche 15 bis 24, wobei zumindest ein Kabel (18) lösbar an dem Prothesenschaft (20) oder der Oberflächenelektrodenanordnung (200) elektrisch kontaktierend mit der Ableiterelektrode (10) oder der Oberflächenelektrode (100) befestigt ist.

26. Prothesensystem nach Anspruch 15, wobei die Stecker (19) mechanisch über ein Sicherungselement (90) gegen ein Lösen gesichert sind.

## Claims

1. Method for configuring a myoelectrically controlled prosthetic system with a prosthesis socket (20) and several lead electrodes (10) for detecting electrical muscle activity, comprising the steps:
- Placing a surface electrode arrangement (200) with a plurality of surface electrodes (100) around the circumference of a residual limb (1)
- Detecting electrical muscle activity in muscles of the residual limb (1) as myoelectric signals by the surface electrodes (100)
- Evaluating the myoelectric signals with regard to their signal quality
- Selecting the control method used to control the prosthetic system based on the evaluation of the signal quality, and
- Fixing the lead electrodes (10) to the prosthesis socket (20).

2. Method according to claim 1, wherein the surface electrode arrangement (200) is designed to be independent from the prosthesis socket (20) and is placed separately from the prosthesis socket (20).

3. Method according to claim 1 or 2, wherein the surface electrode arrangement (200) is placed on the residual limb (1) in a manner that allows it to be shifted and/or twisted.

4. Method according to one of the preceding claims, wherein the surface electrodes (100) are arranged at an equal distance or at least in the main at an equal distance from one another around the residual limb (1).

5. Method according to one of the preceding claims, wherein the signal quality of the myoelectric signals of the surface electrodes (100) is evaluated on the basis of their amplitude, duration, frequency and/or bandwidth.

6. Method according to one of the preceding claims, wherein a selection and definition of a basic setting of the control method is made.

7. Method according to claim 6, wherein the selection of the control method is automatically executed in a control unit (80) on the basis of specific criteria.

8. Method according to claim 6 or 7, wherein pattern recognition is selected as the control method and a basic setting of the pattern recognition method is made which can only be modified and deleted by an authorized person.

9. Method according to claim 6 or 7, wherein a dual-channel control is selected as the control method and the two most distinguishable myoelectric signals are identified and the position of the corresponding electrodes (10, 100) on the stump (1) is indicated in an indicator device.

10. Method according to one of the preceding claims, wherein the position of the surface electrodes (100) is transmitted from the residual limb (1) to the prosthesis socket (20) by means of a positioning aid (31).

11. Method according to one of the preceding claims, wherein the surface electrode arrangement (200) is shifted and/or twisted on the residual limb (1) into an end position in which at least two signals can be detected, and the lead electrodes (10) are fixed on the prosthesis socket (20) correspondingly to the end position.

12. Method according to claim 10, wherein the position of the lead electrodes (10) is displayed on the prosthesis socket (20), projected onto the prosthesis socket (20) or transferred to the prosthesis socket (20) by way of a positioning jig.

13. Method according to one of the preceding claims, wherein the lead electrodes (10) are fixed to the prosthesis socket (20) via a dome bolt connection.

14. Method according to one of the preceding claims, wherein the lead electrodes (10) are connected to a control unit (80) via cables (18) and are mechanically secured from becoming detached by means of a securing element (90).

15. Prosthesis system with a prosthesis socket (20), which can be arranged on a residual limb (1), and several lead electrodes (10) for detecting electrical muscle activities, a control unit that is connected to the lead electrodes (10) and a driven prosthesis component (2), which is fixed to the prosthesis socket (20) and coupled to the control unit (80), with
a surface electrode arrangement (200) with several surface electrodes (100) which can be arranged around the circumference of a residual limb (1), wherein the surface electrode arrangement (200) can be arranged on the residual limb (1) in a manner that allows it to be shifted and/or twisted, **characterized in that** the control unit (80) is connected to the lead electrodes (10) or surface electrodes (100) via plugs(19) such that it can be detached and that an adapter (17) is arranged between the control unit (80) and the plug (19) for converting myoelectric signals, the quality and distinguishability of which are suitable for multichannel control, into two-channel control signals.

16. Prosthesis system according to claim 15, wherein the surface electrode arrangement (200) is designed as a closed, circular strap.

17. Prosthetic system according to claim 15 or 16, wherein the surface electrode arrangement (200) is designed to be elastic in the circumferential direction.

18. Prosthesis system according to one of claims 15 to 17, wherein the surface electrodes (100) are arranged on the surface electrode arrangement (200) at an equal distance or at least in the main at an equal distance from one another in the circumferential direction.

19. Prosthesis system according to one of claims 15 to 18, wherein the surface electrode arrangement (200) has a positioning aid (31) and/or at least one stabilizing element (35) extending in the proximal-distal direction.

20. Prosthesis system according to one of claims 15 to 19, wherein a replaceable cover (70) is arranged on the surface electrode arrangement (200).

21. Prosthesis system according to claim 20, wherein markings are arranged on a positioning aid (31) and/or the cover (70).

22. Prosthesis system according to claim 20 or 21, wherein recesses (71) for surface electrodes (100) are designed to be be provided in the cover (70).

23. Prosthesis system according to one of claims 15 to 22, wherein the surface electrodes (100) and/or lead electrodes (10) are designed as dome bolt (15) with dome nut (14) and are bolted to the surface electrode arrangement (200) or the prosthesis socket (20).

24. Prosthesis system according to claim 23, wherein the dome screw (15) comprises an undercut (153) and the passage spot (23) is sealed against moisture penetration.

25. Prosthesis system according to one of claims 15 to 24, wherein at least one cable (18) is fixed to the prosthesis socket (20) or the surface electrode arrangement (200) such that it can be detached and is in electrical contact with the lead electrode (10) or the surface electrode (100).

26. Prosthesis system according to claim 15, wherein the connectors (19) are mechanically secured against becoming detached by a securing element (90).

## Revendications

1. Procédé de configuration d'un système de prothèse à commande myoélectrique comprenant une emboîture de prothèse (20) et plusieurs électrodes de dérivation (10) pour détecter les activités musculaires électriques, comprenant les étapes consistant à :
- appliquer un ensemble d'électrodes de surface (200) comprenant plusieurs électrodes de surface (100) autour du pourtour d'un moignon de membre (1),
- détecter l'activité musculaire électrique au niveau des muscles du moignon de membre (1) sous forme de signaux myoélectriques par l'intermédiaire des électrodes de surface (100),
- évaluer les signaux myoélectriques en termes de leur qualité,
- sélectionner le procédé de commande, utilisé pour commander le système de prothèse, sur la base de l'évaluation de la qualité des signaux, et
- immobiliser les électrodes de dérivation (10) sur l'emboîture de prothèse (20).

2. Procédé selon la revendication 1,
dans lequel l'ensemble d'électrodes de surface (200) est conçu indépendamment de l'emboîture de prothèse (20) et est appliqué séparément de l'emboîture de prothèse (20).

3. Procédé selon la revendication 1 ou 2,
dans lequel l'ensemble d'électrodes de surface (200) est appliqué sur le moignon de membre (1) de manière mobile en translation et/ou en rotation.

4. Procédé selon l'une des revendications précédentes,
dans lequel les électrodes de surface (100) sont disposées autour du moignon de membre (1) en étant espacées régulièrement ou sensiblement régulièrement les unes des autres.

5. Procédé selon l'une des revendications précédentes,
dans lequel la qualité des signaux myoélectriques des électrodes de surface (100) est évaluée sur la base de leur amplitude, de leur durée, de leur fréquence et/ou de leur bande passante.

6. Procédé selon l'une des revendications précédentes,
consistant à sélectionner et à fixer un réglage de base du procédé de commande.

7. Procédé selon la revendication 6,
dans lequel la sélection du procédé de commande est effectuée automatiquement dans une unité de commande (80) sur la base de critères définis.

8. Procédé selon la revendication 6 ou 7,
dans lequel une reconnaissance de formes est sélectionnée comme procédé de commande, et un réglage de base modifiable et effaçable du procédé de reconnaissance de formes est effectué exclusivement par une personne autorisée.

9. Procédé selon la revendication 6 ou 7,
dans lequel une commande à deux canaux est sélectionnée comme procédé de commande, les deux signaux myoélectriques les plus facilement distinguables sont identifiés, et la position des électrodes correspondantes (10, 100) sur le moignon (1) est affichée sur un dispositif d'affichage.

10. Procédé selon l'une des revendications précédentes,
dans lequel la position des électrodes de surface (100) est transmise du moignon de membre (1) à l'emboîture de prothèse (20) à l'aide d'un dispositif d'aide au positionnement (31).

11. Procédé selon l'une des revendications précédentes,
dans lequel l'ensemble d'électrodes de surface (200) est déplacé en translation et/ou en rotation sur le moignon de membre (1) vers une position finale dans laquelle au moins deux signaux peuvent être détectés, et les électrodes de dérivation (10) sont immobilisées sur l'emboîture de prothèse (20) en correspondance de la position finale.

12. Procédé selon la revendication 10,
dans lequel la position des électrodes de dérivation (10) sur l'emboîture de prothèse (20) est affichée, projetée sur l'emboîture de prothèse (20) ou transmise à l'emboîture de prothèse (20) à l'aide d'un gabarit de positionnement.

13. Procédé selon l'une des revendications précédentes,
dans lequel les électrodes de dérivation (10) sont immobilisées sur l'emboîture de prothèse (20) à l'aide d'un raccord à vis à tête bombée.

14. Procédé selon l'une des revendications précédentes,
dans lequel les électrodes de dérivation (10) sont reliées à une unité de commande (80) par des câbles (18) et sont bloquées mécaniquement à l'aide d'un élément de blocage (90) afin d'empêcher leur détachement.

15. Système de prothèse comprenant une emboîture de prothèse (20) apte à être appliquée sur un moignon de membre (1), et plusieurs électrodes de dérivation (10) pour détecter les activités musculaires électriques, une unité de commande reliée aux électrodes de dérivation (10), et un composant de prothèse (2) à entraîner qui est fixé à l'emboîture de prothèse (20) et couplé à l'unité de commande (80), comprenant
un ensemble d'électrodes de surface (200) comprenant plusieurs électrodes de surface (100) aptes à être appliquées autour du pourtour d'un moignon de membre (1), l'ensemble d'électrodes de surface (200) pouvant être disposé sur le moignon de membre (1) de manière mobile en translation et/ou en rotation,
**caractérisé en ce que**
l'unité de commande (80) est reliée de manière amovible aux électrodes de dérivation (10) ou aux électrodes de surface (100) par l'intermédiaire de fiches (19), et
**en ce qu'**un adaptateur (17) est disposé entre l'unité de commande (80) et la fiche (19) afin de convertir des signaux myoélectriques, dont la qualité et le caractère distinctif sont appropriés pour une commande multicanaux, en signaux de commande à deux canaux.

16. Système de prothèse selon la revendication 15,
dans lequel l'ensemble d'électrodes de surface (200) est conçu comme une bande annulaire fermée.

17. Système de prothèse selon la revendication 15 ou 16,
dans lequel l'ensemble d'électrodes de surface (200) est élastique en direction circonférentielle.

18. Système de prothèse selon l'une des revendications 15 à 17,
dans lequel les électrodes de surface (100) sont disposées sur l'ensemble d'électrodes de surface (200) en étant espacées régulièrement ou sensiblement régulièrement les unes des autres en direction circonférentielle.

19. Système de prothèse selon l'une des revendications 15 à 18,
dans lequel l'ensemble d'électrodes de surface (200) comprend un dispositif d'aide au positionnement (31) et/ou au moins un élément de stabilisation (35) s'étendant dans la direction proximale-distale.

20. Système de prothèse selon l'une des revendications 15 à 19,
dans lequel un revêtement remplaçable (70) est disposé sur l'ensemble d'électrodes de surface (200).

21. Système de prothèse selon la revendication 20,
dans lequel des repères sont disposés sur un dispositif d'aide au positionnement (31) et/ou sur le revêtement (70).

22. Système de prothèse selon la revendication 20 ou 21,
dans lequel des évidements (71) pour des électrodes de surface (100) sont ménagés dans le revêtement (70).

23. Système de prothèse selon l'une des revendications 15 à 22,
dans lequel les électrodes de surface (100) et/ou les électrodes de dérivation (10) sont conçues sous forme de vis à tête bombée (15) avec écrou à tête bombée (14) et sont vissées sur l'ensemble d'électrodes de surface (200) ou sur l'emboîture de prothèse (20).

24. Système de prothèse selon la revendication 23,
dans lequel la vis à tête bombée (15) présente une contre-dépouille (153), et le point de passage (23) est étanchéifié contre la pénétration de l'humidité.

25. Système de prothèse selon l'une des revendications 15 à 24,
dans lequel au moins un câble (18) est fixé de manière amovible à l'emboîture de prothèse (20) ou à l'ensemble d'électrodes de surface (200) en établissant un contact électrique avec l'électrode de dérivation (10) ou avec l'électrode de surface (100).

26. Système de prothèse selon la revendication 15,
dans lequel les fiches (19) sont bloquées mécaniquement à l'aide d'un élément de blocage (90) afin d'empêcher leur détachement.
